# EUROPEAN PATENT APPLICATION

(11) **EP 2 292 246 A1**
(43) Date of publication of application: **09.03.2011**
(21) Application number: 10175701.1
(22) Date of filing: 10.11.2005
(51) Int. Cl.: A61K 35/76, C07K 14/115

(54) **Recombinant Newcastle Disease Virus**

(30) Priority: 12.11.2004 EP 04090432
(62) Divisional of application: 05818947.3
(71) Applicant: Bayer Schering Pharma Aktiengesellschaft, 13353 Berlin (DE)
(72) Inventor: Beier, Rudolf, 13353 Berlin (DE); Bosslet, Klaus, 13465 Berlin (DE); Pühler, Florian, 14089 Berlin (DE); Willuda, Jörg, 16548 Glienicke/Nordbahn (DE)

(57) **Abstract**

The goal of the invention is to increase the therapeutical activity of oncolytic NDV. This issue is solved by a Newcastle Disease Virus comprising a recombinant nucleic acid, wherein the nucleic acid codes for a binding protein that has a therapeutic activity when expressed by the virus-infected tumor cell. Binding proteins belong to the following group: A natural ligand or a genetically modified ligand, a recombinant soluble domain of a natural receptor or a modified version of it, a peptide-ligand, an antibody molecule and derivatives thereof or antibody-like molecules like ankyrin repeat molecules or derivatives thereof.

## Description

### Introduction

The invention refers to a recombinant RNA-virus, preferably a paramyxovirus, preferably Newcastle Disease Virus (NDV) for treatment of diseases, especially for oncolytic tumor treatment. Recombinant viruses are produced that encode binding proteins (antibodies, ankyrin repeat molecules, peptides etc.), prodrug-converting enzymes and/or proteases and lead to the selective expression of these molecules in virus-infected tumor cells. The activity of these binding proteins, prodrug-converting enzymes and/or proteases increases the anti-tumor effect of the virus. Further the invention describes manufacture and the use of such modified viruses for treatment of cancer.

### Description of the State of the Art

### Newcastle Disease Virus

Oncolytic viruses in general for the treatment of tumors are reviewed in Chiocca (2002). Newcastle Disease Virus has been used as an experimental therapeutic agent for more than 40 years and is reviewed by Sinkovics and Horvath (2000). The Newcastle Disease Virus in general is described in the book by Alexander (1988). NDV strain PV701 is being developed as an anticancer treatment for glioblastoma (Lorence et al., 2003). The NDV strain MTH68 has been used as an experimental cancer treatment and has been administered to humans for more than 30 years (Csatary et al., 2004).

In the paper by Stojdl et al. (2003) it is described that in the range of 80% of all tested tumor cell lines, there is a defect in the interferon response following infection with Vesicular Stomatitis Virus (VSV). It may be assumed that a similar percentage of tumor cell lines will be susceptible to infection with NDV because both VSV and NDV are members of the order mononegavirales. It has also been shown that the mechanism of selective replication of NDV in tumor cells is based on a defect in the cellular interferon response against the virus (see e.g. US: 20030044384).

### Recombinant paramyxoviruses

EP-A-0702085 relates to genetically manipulated infectious replicating non-segmented negative-stranded RNA virus mutants, comprising an insertion and/or deletion in an open reading frame, a pseudogen region or an intergenic region of the virus genome.

WO 99/66045 relates to genetically modified NDV viruses obtained from full-length cDNA molecules of the virus genome.

WO 00/62735 relates to a method of tumor treatment comprising administering an interferon-sensitive, replication-competent clonal RNA virus, e.g. NDV.

In WO 01/20989 (PCT/US00/26116) a method for treating patients having tumor with recombinant oncolytic paramyxoviruses is described. The tumor is reduced by administering a replication-competent *Paramyxoviridae* virus. Various methods are described that can be used to engineer the virus genome in order to improve the oncolytic properties.

WO 03/005964 relates to recombinant VSV comprising a nucleic acid encoding a cytokine.

US 6,699,479 describes NDV mutants expressing the V-protein at a reduced level and comprising nucleotide substitutions in an editing locus.

US 2004/0170607 relates to the treatment of melanoma by administering a virus which is not a common human pathogen.

### Genetic manipulation of NDV

NDV can be genetically manipulated using the reverse genetics technology as described e.g. in EP-A-0702 085. For example, it is known to make recombinant NDV constructs comprising additional nucleic acids coding for secreted alkaline phosphatase (Zhao and Peeters, 2003), green fluorescent protein (Engel-Herbert et al., 2003), VP2 protein of infectious bursal disease virus (Huang et al., 2004), influenza virus hemagglutinin (Nakaya et al., 2001) and chloramphenicol acetyl transferase (Huang et al., 2001) (Krishnamurthy et al., 2000). None of these recombinant NDV has been constructed for use in the treatment of human disease. The recombinant NDVs were made to study either basic virology of NDV or to develop vaccine strains for poultry. As parental virus strains served lentogenic strains of NDV. These strains do not have significant oncolytic properties.

### Summary of the Invention

This present invention relates to an RNA virus, e.g. an NDV having increased oncolytic activity. More particularly, the invention relates to an RNA virus, particularly a Newcastle disease virus comprising a recombinant nucleic acid having the therapeutical relevance in treatment of cancer, wherein the nucleic acid codes for a binding protein, a prodrug-converting enzyme and/or a protease that have a therapeutic activity when expressed by the virus-infected tumor cell.

More preferred are recombinant oncolytic viruses, e.g. NDVs comprising one ore more transgenes, wherein the transgene(s) is / are coding for a binding protein, a prodrug-converting enzyme and/or a protease. Combinations of different specificities are possible.

Further, the invention relates to the nucleocapsid of the recombinant virus as indicated above, comprising viral RNA complexed with capsid proteins or to the viral RNA and/or an RNA complementary to the viral RNA in its isolated form.

Furthermore, the invention relates to a DNA, e.g. a cDNA encoding the viral RNA and/or an DNA complementary to the viral RNA. Furthermore, the invention relates to the prevention or treatment of tumor diseases.

### Detailed description of Preferred Embodiments:

### Viruses

The invention generally relates to RNA viruses, preferably negative strand RNA viruses, more preferably such viruses that have both oncolytic properties and can be genetically engineered. Such viruses are:
- paramyxoviruses, preferably Newcastle Disease Virus (NDV), measles virus, mumps virus, Sendai virus;
- orthomyxoviruses, preferably influenza virus;
- rhabdoviruses, preferably vesicular stomatitis virus.

Therefore, the subject of the present invention is a recombinant oncolytic RNA virus comprising a nucleic acid with at least one transgene, wherein the nucleic acid of this transgene(s) codes for a binding protein that has a therapeutic activity when expressed by the virus-infected tumor cell. Another subject of the present invention is a recombinant oncolytic RNA virus comprising a nucleic acid with at least one transgene, wherein the nucleic acid of this transgene(s) codes for a prodrug-converting enzyme that has a therapeutic activity when expressed by the virus-infected tumor cell, preferably in combination with the corresponding prodrug.

Yet another subject of the present invention is a recombinant oncolytic RNA virus comprising a nucleic acid with at least one transgene, wherein the nucleic acid of this transgene(s) codes for a protease that has a therapeutic activity when expressed by the virus-infected tumor cell.

Preferably the virus of the present invention exhibits a tumor-selective infection that leads to a tumor-selective expression of the encoded transgene.

The recombinant oncolytic virus of the present invention may carry at least one transgene gene independently selected from transgenes coding for binding proteins, prodrug-converting enzymes, and/or proteases.

It is preferred that the virus of the present invention is a negative strand RNA virus, more preferably a paramyxovirus.

In the context of the present invention, a nucleic acid with at least one transgene is a nucleic acid comprising a gene which is heterologous to the oncolytic RNA virus on which the recombinant RNA virus of the present invention is based. The term "heterologous" refers to the complete gene or a part thereof, which may be the coding region of the gene or a part thereof.

The heterologous gene may be an artificial sequence or may be obtained from natural sources or by recombination of at least two sequences selected from sequences obtained from natural sources and/or artificial sequences. "Natural sources" include animals such as mammals, plants, fungi, and microorganisms such as bacteria, protozoa and viruses, which may be different from other oncolytic RNA viruses of the present invention. The transgene may also encode for a fusion protein. Mammals include humans and mice.

In an especially preferred embodiment, the virus of the present invention is a Newcastle Disease Virus, (NDV), most preferred is NDV strain MTH68. The NDV may be a lentogenic, mesogenic or velogenic strain. Especially preferred are mesogenic or velogenic NDV strains. The virus is preferably replication competent.

### Genetic manipulation of viruses

Methods for genetically manipulating RNA viruses are well known as stated above. Further, genetic manipulation of oncolytic viruses is reviewed e.g. in Bell et al. (2002). RNA viruses as virotherapy agents are reviewed in Russell (2002). The content of any of these documents is herein incorporated by reference.

### Binding proteins

In the oncolytic recombinant RNA virus of the present invention, at least one transgene may code for a binding protein.

Binding proteins are proteins, which, when expressed in a target cell, are capable of binding to a component of said cell and/or a neighbouring cell. Preferably, binding proteins are proteins which bind to intracellular components.

In a preferred embodiment, binding proteins belong to the following group: a natural ligand or a genetically modified ligand, a recombinant soluble domain of a natural receptor or a modified version of it, e.g. a peptide- or polypeptide-ligand, an antibody molecule and fragments and derivatives thereof or an antibody-like molecule and derivatives thereof.

An incomplete review of high-affinity binding frameworks is given by Ladner and Ley (2001).

The binding proteins as described above might be of human, murine or closely related origin or a chimeric version, i.e. a protein which may be a fusion protein comprising sequences from different species, e.g. human and mouse.

The recombinant binding molecules based on the description above can be monomeric, dimeric, trimeric, tetrameric or multimeric and bispecific or multispecific.

The preferred binding proteins are selected from binding proteins having a therapeutic activity.

A natural ligand as described above can be a growth factor or a peptide. A genetically modified ligand may be an analogue of a naturally occurring growth factor or peptide.

Recombinant soluble domains of a natural receptor or modified versions of it as described above are recombinantly expressed soluble extracellular domains of a cell-surface receptor and/or fragments of it, a recombinantly expressed soluble extracellular domain of a cell adhesion molecule and/or fragments thereof.

Antibody molecules as mentioned above may be monoclonal immunoglobulin antibodies of any known specificity and isotype, fragments thereof and/or fragments thereof fused to effector proteins. The antibody molecules may be chimeric, humanized or human antibodies. Antibody fragments contain at least one antigen-binding domain of an antibody. Antibody fragments have been described extensively in the literature (reviewed eg. in Allen (2002), herein incorporated by reference). Preferred examples are single-chain Fv fragments, Fab fragments, F(ab2'), domain-deleted versions called minibodies, and other immunoactive portions, fragments, segments and other smaller or larger partial antibody structures wherein the latter possess sufficient targeting properties or immunological stimulatory or inhibitory activity so as to be therapeutically useful within the methods of the present invention.

Such antibodies may be derived from hybridoma cloning experiments by use of transgenic mice or from phage display selections, ribosome display selections, or colony filter screening of antibody libraries containing human antibody sequences or related methodologies.

Binding proteins with antibody like properties as described above may be genetically modified proteins or domains of it in which one or more peptide loops are randomized on the level of amino acids in such a way that high affinity binding molecules with high specificity can be enriched against any antigen from libraries of such molecules by phage display, ribosome display, colony filter screen or related methodologies. The selected proteins usually have high thermal and thermodynamic stability and are well expressed in recombinant expression systems such as E.coli, yeast, insect and mammalian expression system. Examples for such binding proteins with antibody like properties are ankyrin repeat proteins as described in Binz et al. (2004), the lipocalins as described in Skerra (2000), the gamma-crystallins as described in DE199 32 688.6, the modified protein A scaffold (affibodies) as described in Hogbom et al. (2003), or Nord et al. (2000) or the fibronectin framework and others. Antibody-like molecules can be monomeric or repetitive molecules either constructed as single-chain molecules or as multichain molecules wherein the antibody-like molecule possesses sufficient targeting properties or immunological stimulatory or inhibitory activity so as to be therapeutically useful within the methods of the present invention.

### Binding molecules with additional function

The binding protein may be a fusion protein comprising at least one binding domain, e.g. from an antibody, and a heterologous domain. "Heterologous" has the meaning as discussed above in the context of heterologous genes.

The binding proteins described above are able to deliver a payload to a disease specific site (e.g. a tumor) as a so called intrabody or as extracellular available binding protein. The delivered payload can be a heterologous domain, e.g. a toxin such as human RNAse (De Lorenzo et al., 2004) (Zewe et al., 1997) *Pseudomonas* exotoxin (Chaudhary et al., 1989) (Kreitman and Pastan, 1995) (Batra et al., 1992) , Diphtheria toxin (Kreitman et al., 1993) (Chaudhary et al., 1990) (Batra et al., 1991), or an enzyme such as beta-galactosidase, beta-glucuronidase (Roffler et al., 1991) (Wang et al., 1992) (Bosslet et al., 1992), beta-glucosidase (Rowlinson-Busza, 1992), carboxypeptidase, (Antoniw et al., 1990), (Bagshawe et al., 1988), beta-lactamase with therapeutic efficacy, or an immune-stimulatory protein with cytokine activity such as IL-2, IL-12, TNF-alpha, IFN-beta or GM-CSF (see eg. review by Allen (2002).

In another example the binding proteins described above have themselves **antagonistic or agonistic efficacy** which is therapeutically useful. Examples for antagonistic/blocking binding molecules are the VEGF inhibitory antibody Avastin (Ferrara et al., 2004), the HER2/neu receptor blocking antibody Herceptin (Noonberg and Benz, 2000) or the EGF-receptor blocking antibody Erbitux (Herbst and Langer, 2002). Agonistic binding proteins can be binding proteins which induce for example apoptosis (Georgakis et al., 2005) or have regulatory activity on DNA, RNA or proteins (e.g. induce transcription, stabilize proteins). The review by (Adams and Weiner, 2005) describes various therapeutic antibodies that could also be incorporated into an oncolytic virus

### Prodrug-converting enzymes

In the oncolytic recombinant RNA virus of the present invention, at least one transgene may code for a prodrug-converting enzyme.

A prodrug is a derivative or a precursor of a therapeutically active compound, which can be enzymatically converted into the active compound. Prodrug-converting enzymes are enzymes capable of converting a prodrug into the therapeutically active drug.

Therefore subject of the present invention is a pharmaceutical composition comprising a recombinant oncolytic virus of the present invention, a virus genome of the present invention, a virus antigenome of the present invention, and/or a DNA molecule of the present invention as an active ingredient optionally together with pharmaceutically acceptable carriers, diluents and/or adjuvants, which virus, virus genome, antigenome and/or DNA molecule comprises at least one transgene encoding for a prodrug-converting enzyme. The pharmaceutical composition may further comprise a prodrug which can be converted into a therapeutically active compound by the prodrug-converting enzyme encoded by the virus, virus genome, antigenome and/or DNA molecule. The pharmaceutical composition may be suitable for treatment and/or alleviation of a proliferative disorder.

The prodrug may be formulated in a single composition with the recombinant oncolytic virus of the present invention, a virus genome of the present invention, a virus antigenome of the present invention, and/or a DNA molecule of the present invention as an active ingredient, or may be formulated in a composition distinct from the oncolytic virus formulation.

If the oncolytic recombinant RNA virus of the present invention encodes for a prodrug-converting enzyme, the oncolytic virus of the present invention causes selective expression of the prodrug-converting enzyme in a virus-infected target cell (in particular a tumor cell) which is usually not or not sufficiently expressing the prodrug converting enzyme. Thus, during treatment of a subject in need thereof, the prodrug is specifically converted into the pharmaceutical active compound in a target cell, in particular in a tumor cell, but may essentially not be converted into the therapeutically active compound in a non-target cell, in particular in a healthy cell of the subject to be treated. Thus, undesired side-effect of the therapeutically active compound are reduced compared with treatment of the therapeutically active compound alone.

In the context of the present invention, the prodrug may be a derivative or a precursor of a therapeutically active compound suitable for treatment and/or alleviation of a proliferative disorder, which prodrug can be converted by a prodrug converting enzyme. The prodrug may be a compound known by a person skilled in the art. Derivatives and/or precursors are known by a person skilled in the art.

It is preferred that the prodrug is essentially pharmaceutically inactive and/or nontoxic.

Examples of prodrug-converting enzymes of the present invention are beta-glucuronidase, beta-galactosidase, beta-glucosidase, carboxypeptidase, beta-lactamase, D-amino acic oxidase. Further examples are known by a person skilled in the art.

It is preferred that the prodrug-converting enzyme is essentially not expressed in non-tumor cells.

The prodrug-converting enzyme may be obtained from an organism selected from mammals, plants, fungi, and microorganisms such as bacteria, protozoa and viruses.

A most preferred combination of the prodrug-converting enzyme and a prodrug is E. coli beta-glucuronidase and a prodrug which can be converted by beta-glucuronidase into an active cytotoxic compound. An examle is HMR1826 (doxorubicin-glucuronide) which can be converted into doxorubicin which is a known compound for treatment of cancer.

Another subject of the present invention is a method for treatment of a proliferative disease, in particular a hyperproliferative disease, such a cancer, comprising administering in a pharmaceutically effective amount to a subject in need thereof
(a) a recombinant oncolytic virus of the present invention, a virus genome of the present invention, a virus antigenome of the present invention, and/or a DNA molecule of the present invention comprising at least one transgene encoding for a prodrug-converting enzyme, and
(b) a prodrug suitable for treatment of the proliferative disease, which prodrug can be converted into a pharmaceutically active compound by the prodrug-converting enzyme of (a).

The method may comprise the administration of a single pharmaceutical composition comprising both components (a) and (b), or may comprise the administration of two distinct pharmaceutical compositions, one of which comprises component (a) and the other comprises (b).

### Proteases

In the oncolytic recombinant RNA virus of the present invention, at least one transgene may code for a protease.

Therefore subject of the present invention is a pharmaceutical composition comprising a recombinant oncolytic virus of the present invention, a virus genome of the present invention, a virus antigenome of the present invention, and/or a DNA molecule of the present invention as an active ingredient optionally together with pharmaceutically acceptable carriers, diluents and/or adjuvants, which virus, virus genome, antigenome and/or DNA molecule comprises at least one transgene encoding for a protease. The pharmaceutical composition may be suitable for treatment and/or alleviation of a proliferative disorder.

If the oncolytic recombinant RNA virus of the present invention encodes for a protease, the oncolytic virus of the present invention causes selective expression of the protease in a virus-infected target cell (in particular a tumor cell) which is usually not or not sufficiently expressing the protease. Thus, during treatment of a subject in need thereof, the protease may irreversibly cleave a target polypeptide in a target cell, thereby inhibiting proliferation and/or growth of the target cell or killing the target cell, but may essentially not cleave the target molecule in a non-target cell, in particular in a healthy cell of the subject to be treated. By this strategy, undesired side-effects of protease treatment are reduced.

It is preferred that the protease is a sequence-specific protease. More preferred is a protease specifically cleaving a target polypeptide. The protease may either be of natural origin and may be derived from any species or it may be engineered. Amino acid sequences suitable for a specific cleavage of a predetermined target polypeptide can be determined by a person skilled in the art, e.g. on the basis of publicly available sequence databases. US 2005-0175581 and US 2004-0072276 describe the generation of protein-engineered proteases with a predetermined substrate specifity. These two documents are herein included by reference.

The target molecule of the protease may be any target molecule as described below for targets of binding proteins.

Another subject of the present invention is a method for treatment of a proliferative disease, in particular a hyperproliferative disease, such a cancer, comprising administering in a pharmaceutically effective amount to a subject in need thereof a recombinant oncolytic virus of the present invention, a virus genome of the present invention, a virus antigenome of the present invention, and/or a DNA molecule of the present invention comprising at least one transgene encoding for a protease.

The transgene of the present invention may encode a fusion protein of a prodrug-converting enzyme as defined above, a binding molecule as defined above and/or a protease as defined above. Especially preferred is a fusion protein of a prodrug-converting enzyme and a binding molecule or a fusion protein of a protease and a binding molecule.

### Therapeutic Applications

The present invention shows for the first time that a transgene coding for a binding protein, a prodrug-converting enzyme and/or a protease may be functionally expressed in oncolytic virus-infected tumor cells. Thus, the present invention relates to a pharmaceutical composition which comprises as an active ingredient a virus as indicated above, a nucleocapsid of the virus, a genome of the virus or a DNA molecule encoding the genome and/or the antigenome of the virus, optionally together with pharmaceutically acceptable carriers, diluents and/or adjuvants.

The pharmaceutical composition may be provided as a solution, suspension, a lyophilisate or in any other suitable form. In addition to the active ingredient, the composition may comprise carriers, buffers, surfactants and/or adjuvants as known in the art. The composition may be administered e.g. orally, topically, nasally, pulmonally or by injection locally or intravenously. The pharmaceutical composition is administered in a pharmaceutically effective amount depending on the type of disorder, the patient's condition and weight, the route of administration etc. Preferably 10⁹ to 10¹² virus particles, 10⁸ to 10¹¹, 10⁷ to 10¹⁰, or 10⁶ to 10⁹ virus particles are administered per application. The oncolytic therapy may be optionally combined with other tumor therapies such as surgery, radiation and/or chemotherapy such as cyclophosphamide treatment and/or hyperthermia treatment.

According to the present invention, a recombinant oncolytic paramyxovirus can express a soluble binding protein, a prodrug-converting enzyme and/or a protease that may remain either in the infected cell or may be secreted, such as an antibody, an antibody fragment, an ankyrin repeat protein or another binding molecule as specified below. It has especially not been described that such a protein can be expressed by an oncolytic strain of an RNA virus, e.g. of a Newcastle disease virus.

As an example NDV, the strain MTH68 was chosen in the present application because it has an inherent oncolytic property with promising data from experimental clinical treatments of patients (Sinkovics and Horvath, 2000). In principle, however, most NDV strains with multibasic fusion protein cleavage sites may be used as oncolytic agents for the treatment of tumors. The reverse genetics technology is applicable to all strains.

Binding proteins as described above have been demonstrated to be of high therapeutic potential.

The combination of oncolytic NDV with therapeutic binding proteins, prodrug-converting enzymes and/or proteases of the above described properties will have additional or even synergistic efficacy of two therapeutical principles. The oncolytic self-replicating virus targets the binding protein drug, the prodrug-converting enzyme and/or the protease to the preferred site of action where it is expressed in situ in high local concentrations. Such protein expression is expected to be very selective and the binding protein, the prodrug-converting enzyme and/or the protease with its respective mode of action will add to the intrinsic therapeutic oncolytic activity of the NDV. Based on the replication competent nature of the used virus and the selective replication in tumor cells the amount of expressed transgene [binding protein, the prodrug-converting enzyme and/or protease] is expected to be roughly proportional to the mass of the tumor.

**Antibody molecules or antibody like molecules** or derivatives thereof are ideal binding proteins to be used with the NDV-system. Antibody molecules have been the subject of intensive research and technologies are now available to generate antibody molecules which are non-immunogenic, very selective and of high affinity. The local expression of antibody molecules at high concentrations lead to very significant agonistic or antagonistic efficacy or efficient targeting of effector molecules with reduced toxicity profile compared to standard therapy.

The use of antibody-like molecules in the NDV system is expected to be even superior. These molecules are designed for selective high affinity binding with very high thermal stability and yield compared to normal antibodies. In the case of the **ankyrin-based** antibody-like molecules the repetitive nature of the molecule can be finetuned according to the respective target for optimized targeting, binding, inhibition or activation. Also different binding specificities can be combined within one ankyrin molecule, exploiting the possibility of joining in one ankyrin-repeat molecule several units with different binding specificities. This modular structure allows the multivalent binding of greater protein surfaces than it is possible for antibodies, which can be extremely important in blocking protein-protein interactions. The modular structure can also be exploited to block several effectors with only one single blocking ankyrin-repeat-protein.

Since the ankyrin-repeat-molecules are extremely stable even under reducing condition these molecules can be designed to target proteins inside the cell ("Intrabody").

Also possible is the use of libraries of binding protein-coding sequences with NDV for in vivo target identification.

Possible **targets** for binding molecules or/and proteases can be all structures of a target cell or of the extracellular matrix surrounding the target cell which can be recognized by the described binding proteins or/and proteases and which are relevant to a certain type of pathological phenotype. These can be structural proteins, enzymes, growth factors, growth factor receptors, integrins, transcription factors etc.

Even targets that are not drugable by small molecules (protein-protein interactions, DNA-binding etc.) can be addressed by this invention.

The combination of the oncolytic NDV and therapeutic binding proteins, prodrug-converting enzymes and/or proteases as described above are envisaged for the treatment of inflammatory disease e.g. rheumatoid arthritis and of cancer.

For the **treatment of cancer** all **pathways** which contribute to the development of cancer can be targeted. These pathways are: self-sufficiency in growth signals, insensitivity to growth-inhibitory (antigrowth) signals, evasion of apoptosis, limitless replicative potential, sustained angiogenesis, and tissue invasion and metastasis. A summary of these pathways is given in (Hanahan and Weinberg, 2000). Signaling pathways that are involved in the tumorigenesis process and can be targeted by the described approach are the receptor tyrosine kinase pathway (RTK) pathway, RB and p53 pathway, apoptosis pathway, APC pathway, HIF1 pathway, GLI pathway, PI3K pathway and the SMAD pathway. A detailed description of these signaling pathways are given in Vogelstein and Kinzler (2004).

Other signaling pathways where described binding proteins could interfere with are the ras, Wnt and Hedgehog pathway, where for example protein protein interactions can be blocked.

Examples of binding proteins intervening beneficially in the above described pathways in cancer cells are:
- blocking proteins of autonomous active growth factor receptors (eg. EGFR, Met)
- competitive binders for growth factors (antagonists)
- blocking proteins for Rb-phosphorylation
- blocking proteins for E2F-dependent transcription
- stabilizers for p53
- antagonistic binders for antiapoptotic proteins (e.g. Bcl-2)
- antagonistic binders for cyclins
- antagonistic binders for Ras effectors (eg. GEFs)
- antagonistic binders for hypoxia induced proteins (e.g. HIF1α)
- inhibitors of transcription factors that interfere with dimerization or DNA-binding or cofactor binding (eg. Myc/Max)
- inducers of differentiation
- inhibitors of smad signalling/translocation
- inhibitors of cellular adhesion interactions (cadherins, integrins, eg. α5β1, αvβ3)
- inhibitors of enzymes that degrade the extracellular matrix (eg. MMPs)
- antagonistic binders for proangiogenic ligands (eg. soluble VEGF-R)
- inhibitors of mitotic kinases (eg. PIk-1 )
- antagonistic binders to proangiogenic receptors
- inhibitors of scaffold complex formation (eg. KSR/Ras)
- inhibitors of translation initiation (eIF4E, EIF2a)

The protease of the present invention, the prodrug-converting enzyme and/or the therapeutically active compounds derived from prodrugs of the present invention by the prodrug-converting enzyme may also beneficially intervene in the above described pathways of cancer cells.

### Definitions

### Newcastle Disease Virus:

Paramyxoviruses contain single-stranded RNA genomes of negative polarity having genomes of 15-19 kb in length (wild-type) and the genomes contain 6-10 genes. The viral envelope is formed by the surface glycoproteins and a membrane part derived from the host cell. The surface glycoproteins (F and HN or H or G) mediate entry and exit of the virus from the host cell. The nucleocapsid is inside the envelope and contains the RNA genome and the nucleocapsid protein (NP), phospho- (P) and large (L) proteins responsible for intercellular virus transcription and replication. The matrix (M) protein connects the viral envelope and the nucleocapsid. In addition to these genes encoding structural proteins, *Paramyxoviridae* may contain "accessory" genes which may be additional transcriptional units interspersed with the genes mentioned above. The accessory genes are mostly ORFs that overlap with the P gene transcriptional unit. A comprehensive description of paramyxoviridae can be found in (Lamb, 2001).

NDV is the prototypic member of the genus *Avulavirus* in the family *Paramyxoviridae* belonging to the order *Mononegavirales.* The viral genome is a single-stranded negative-sense RNA coding for six major proteins: the nucleocapsid protein (NP), phosphoprotein (P), matrix protein (M), fusion protein (F), hemagglutinin protein (HN), and the polymerase protein (L). By editing of the P protein mRNA, one or two additional proteins, V (and W), are translated.

NDV strains are classified on their pathogenicity for chicken as velogenic strains (highly virulent) leading to acute lethal infection of chicken of all ages, mesogenic isolates (intermediate virulence) that are only lethal in young chicks, and lentogenic strains (nonvirulent) manifested in a mild or unapparent form of the disease. Classification of NDV isolates in velo-, meso- or lentogen is determined by the mean death time (MDT) of the chicken embryo in 9 day-old embryonated eggs after inoculation with the minimum lethal dose to kill the embryo. One of the determinants of NDV virulence seems to be the cleavage site of the precursor F protein.

NDV is in detail characterized in Alexander (1988) and Lamb (2001).

### Recombinant virus

Recombinant virus means a virus that has an engineered defined alteration in its genomic RNA sequence. This alteration may be one or more insertions, deletions, point mutations or combinations thereof.

A recombinant RNA virus of the present invention may comprise the full genomic sequence of a natural (unmodified) RNA virus or a sequence derived thereof and may additionally comprise at least one recombinant transcriptional cassette. The at least one transcriptional cassette may be located in between two genes (transcriptional units) of the viral genome. In this case, the at least one transcriptional cassette is flanked by transcriptional start and stop sequences. The at least one transcriptional cassette may also be located within a transcriptional unit of the viral genome. In this case, no additional transcriptional start and stop sequences are required.

The at least one transcriptional cassette may comprise restriction sites, such as Pacl or/and Ascl, which may be unique. If two transcriptional cassettes are present, they may comprise different restriction sites.

It is preferred that the RNA virus of the present invention comprises one or two recombinant transcriptional cassettes.

In the at least one transcriptional cassette of the present invention, there is a transgene located, which may encode for a binding protein,a prodrug-converting enzyme and/or a protease as described above.

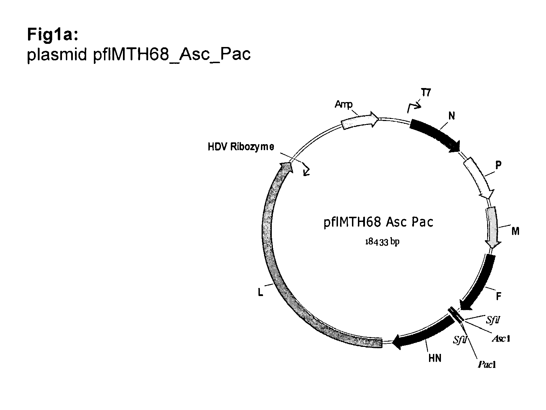

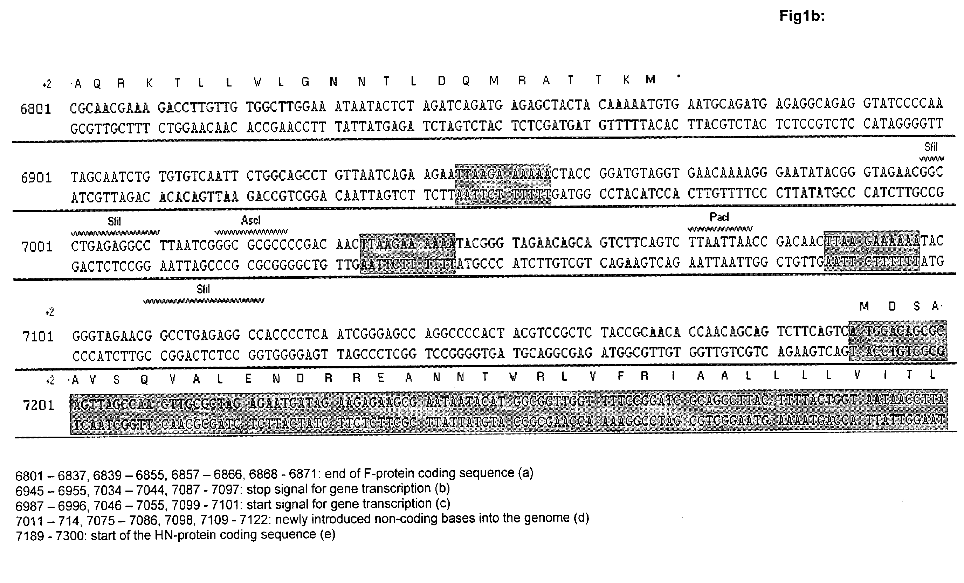

Any intergenic region between each of two genes (transcriptional units) of the viral genome is suitable for introducing the at least one recombinant transcriptional cassette. If more than one recombinant transcriptional cassette is present, they may be located in the same or different intergenic regions. Figure 1 describes an example of two recombinant transcriptional cassettes within one intergenic region. It is preferred that at least one recombinant transcriptional cassette is located between the viral F and HN genes, in particular if the RNA virus of the present invention is a recombinant Newcastle Disease Virus.

There is no known upper limit for the size of the genome of *Paramyxoviridae.* Therefore, there is no upper limit for the number and size of transgenes introduced into the recombinant RNA virus of the present invention. It is preferred that the transgene has a size of up to about 10 kb, more preferred up to about 5 kb, most preferred up to about 2 kb.

The recombinant RNA virus of the present invention preferably carries up to five transgenes, more preferably up to four transgenes, even more preferably up to three transgenes, most preferably one or two transgenes. If the recombinant virus of the present invention carries at least two transgenes, they may be identical or different. The recombinant RNA virus of the present invention may carry more than one copy of a particular transgene, in particular two, three, four of five copies.

In the expression (including transcription of the viral RNA into mRNA and translation of the mRNA) of the transgene, expression control sequences such as transcriptional start and stop sequences and sequences controlling the translation are used. The expression control sequences of an RNA virus may be used which may be the RNA virus on which the recombinant RNA virus of the present invention is based. In particular, transcriptional start and stop sequences may be obtained from an RNA virus. Expression control sequences may also be obtained from a target cell, in particular sequences controlling the translation and/or protein transport.

Due to the replication mechanism of *Paramyxoviridae,* the genomic or antigenomic RNAs usually do not appear as naked RNAs. The genomic and antigenomic RNAs are assembled with the nucleoprotein. Therefore, a further subject of the present invention is a nucleocapsid of a recombinant oncolytic RNA virus of the present invention. The nucleocapsid comprises the RNA molecule encoding the genome or/and the antigenome of the RNA virus and the nucleocapsid protein. The nucleocapsid may also comprise the polymerase protein L or/and the phosphoprotein P.

Also subject of the present invention is the anti-genome of the genome of the present invention as described above.

A further aspect of the present invention is a DNA molecule encoding the genome and/or the anti-genome of a recombinant oncolytic RNA virus of the present invention. The DNA molecule may be a plasmid. The DNA molecule of the present invention can be used for genetically engineering the RNA virus of the present invention. Further, the DNA molecule may be used for producing the RNA virus of the present invention. Therefore, the DNA molecule may be operatively linked to a transcriptional control sequence e.g. a prokaryotic or eukaryotic transcription control sequence.

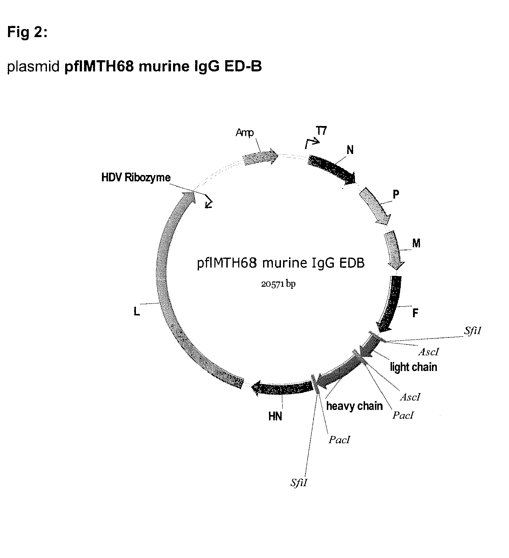

An example of DNA molecules of the present invention is pfIMTH68 murine IgG EDB (Fig. 2).

The genome, antigenome, nucleocapsid and/or DNA molecule of the present invention may comprise at least one transgene which may be located within the transcriptional cassette as described above. As discussed above, the transgene may encode for a binding protein, a prodrug-converting enzyme and/or a protease.

Another aspect of the present invention is a method for producing a recombinant oncolytic RNA virus comprising expressing a DNA molecule encoding the genome and/or the anti-genome of a recombinant oncolytic virus of the present invention.

A further aspect of the present invention is a cell comprising the recombinant oncolytic virus of the present invention, a virus genome of the present invention, a virus anti-genome of the present invention and/or a DNA molecule of the present invention. The cell may be a prokaryotic cell or a eukaryotic cell. The cell may be a cell line, in particular a mammalian cell line, more particularly a human or murine cell line. The cell may be used in the method of the present invention for producing the RNA virus of the present invention. Suitable systems for transcribing a DNA molecule are known by a person skilled in the art, e.g. in prokaryotic systems such as *E*. *coli* or eukaryotic systems such as HeLa or CHO.

Yet another aspect is an oncolytic RNA virus, a genome or anti-genome thereof or a DNA molecule comprising the full set of genes of *Paramyxoviridae* or a set of genes of *Paramyxoviridae* in which at least one gene or intergenic region is genetically modified, and further comprising at least one recombinant transcriptional cassette as described above. Such a virus, genome or antigenome or DNA molecule may be used for the manufacture of a medicament and/or treatment of cancer. Such RNA virus, genome, anti-genome or DNA molecule is suitable for constructing a recombinant *Paramyxoviridae* virus, in particular a recombinant Newcastle Disease Virus by genetic engineering techniques in order to introduce a recombinant sequence into the transcription cassette. For this purpose, the at least one transcription cassette may comprise a restriction site. If more than one transcription cassettes are present, the unique restriction sites of the transcriptional cassettes may be different. An example is plasmid pfIMTH68_Asc_Pac of Fig. 1.

### Therapeutical relevance:

**Treatment of Cancer** means inhibition of tumor growth, preferably the killing of the tumor cells or the blocking of proliferation in a time gap by infection. NDV replicates selectively in tumor cells.

**The virus of the present invention can be used to treat proliferative disorders, in particular hyperproliferative disorders. Preferably neoplasms** can be treated with the described virus, preferably cancers from the group consisting of lung, colon, prostate, breast and brain cancer can be treated.

More preferably a solid tumor can be treated.

More preferably a tumor with low proliferation rate can be treated.

Examples of tumors with low proliferation rate are prostate cancer or breast cancer.

More preferably a brain tumor can be treated.

More preferably a glioblastoma can be treated.

### Manufacture of the recombinant RNA virus

The recombinant RNA virus of the present invention, in particular the recombinant NDV, can be constructed as described in Romer-Oberdorfer et al. (1999). The construction of the new nucleic acid sequences is on the level of the cDNA which then is translated into RNA within a eucaryotic cell using the following starting plasmids:
pCITE P, pCITE N, pCITE L, pX8δT fINDV

NDV can be any strain of Newcastle Disease Virus, more preferred a strain that is oncolytic in its wildtype form.

The plasmid pX8δT is described in EP0702085 (Conzelmann KK).

The recombinant RNA virus of the present invention, in particular the recombinant NDV, can be recovered initially from T7 polymerase expressing cells, eg. BHK T7 cells or transiently with T7 polymerase transfected CHO cells. It can be amplified in cells like 293, CEC32, HT29 or A431. It can also be amplified in the allantoic fluid of embryonated chicken eggs.

The recombinant RNA virus, in particular the recombinant NDV, is stored under the following conditions. The recombinant RNA-virus, in particular NDV is stable in 5% D-mannitol /1% (w/v) L-lysine / pH 8,0 or standard cell culture medium.

At -20°C for up to one month.

At -80°C for up to 10 years.

### Use of the recombinant NDV as a Medicament

The recombinant RNA virus of the present invention, in particular the purified recombinant NDV according to the invention can be used as a medicament, because it shows pharmacological effects.

The recombinant RNA virus of the present invention, in particular the NDV of the invention is a medicament especially for prevention and/or treatment of cancer, especially for prevention and/or treatment of lung cancer, prostate cancer, brain cancer, colon cancer, breast cancer.

The invention comprises the recombinant RNA virus of the present invention, in particular the NDV of the invention as a medicament combined with pharmaceutically acceptable carrier and diluents. Such carrier and diluents are described in Remington's Pharmaceutical Science, 15^{th} ed. Mack Publishing Company, Easton Pennsylvania (1980). The used virus titers may be in the range of 10⁹ to 10¹² pfu per dose, in a range of 10⁸ to 10¹¹ pfu, in a range of 10⁷ to 10¹⁰ pfu or in a range of 10⁶ to 10⁹ pfu dependent on the indication of treatment.

Therefore, another subject of the present invention is a pharmaceutical composition comprising a recombinant oncolytic virus of the present invention, a virus genome of the present invention, or a DNA molecule of the present invention together with pharmaceutically acceptable carriers, diluents and/or adjuvants. The pharmaceutical composition of the present invention may be used for the prevention or/and treatment of a proliferative disorder, such as cancer.

The pharmaceutical composition of the present invention may comprise an emulsion of the recombinant oncolytic RNA virus of the present invention, in particular the NDV of the invention and may be administered by inhalation, intravenous infusion, subcutaneous injection, intraperitoneal injection or intratumoral injection.

Yet another subject of the present invention is a method for the prevention or/and treatment of a proliferative disorder, in particular cancer, comprising administration to a subject in need thereof a pharmaceutically effective amount of the pharmaceutical composition of the present invention. A pharmaceutically effective amount is a titre of the oncolytic RNA virus of the present invention, in particular the NDV of the present invention, the virus genome of the present invention, or the DNA molecule of the present invention which cures or suppresses the disease.

For the therapeutic effect the acceptable dosis is different and depends for example from the construct, the patient, the ways of administration and the type of cancer.

It is preferred that the subject (the patient) is a mammal, more preferably a human patient.

Yet another aspect of the present invention is the use of the recombinant RNA virus of the present invention, in particular the NDV of the invention, the virus genome of the present invention, or the DNA molecule of the present invention for manufacture of a medicament for treatment of cancer.

The present invention is further illustrated by the following Figures and Examples.

### Figure legends

Figure 1 describes plasmid pfIMTH68_Asc_Pac comprising the full genome of NDV and two transcriptional cassettes comprising the unique restriction site Ascl or Pacl, respectively. The nucleotide sequence describes the intergenic region between F and HN comprising two recombinant transcriptional cassettes (SEQ. ID. NO: 5).
Figure 2 describes plasmid pfIMTH68 murine IgG ED-B, which is based on pfIMTH68_Asc_Pac and comprises sequences encoding the light chain and the heavy chain of anti-ED-B IgG antibody MOR03257 (see claim 42 of German Patent Application DE10 2004 05 0101.7-43; Seq ID No. 77). The light chain and the heavy chain are each inserted in one of the transcriptional cassettes.
Figure 3 describes that infection of CHO or HT29 cells with NDV MTH146 containing the genes for the anti-ED-B IgG antibody MOR03257 leads to expression of the antibody into the cell supernatant. 24 hours post infection, a level of about 20% of the maximal expression level is reached. After about 72 hours post infection, maximal expression of the antibody is reached. As a control, cells were infected with MTH115 (containing the gene for beta-glucuronidase). In these cells, no IgG was detected.
Figure 4 demonstrates by immunofluorescence that cells infected with MTH146 exhibit a staining pattern indistinguishable from the staining with the recombinant antibody MOR03257.
Figure 5 demonstrates by immunohistochemistry that the supernatant from MTH146 infected cells yields an undistinguishable staining pattern compared with purified αED-B antibody MOR03257 expressed from stably transfected 239 cell in tumor vasculature where ED-B is present.
Figure 6 demonstrates that intravenous injection of MTH87 leads to expression of GFP in the tumor in a mouse xenograft model.
Figure 7 describes expression of β-glucuronidase in MTH115 infected Hela cells.
Figure 8 describes expression levels of β-glucuronidase in MTH115-infected cell lines.
Figure 9 and Figure 10 describe synergistic effect of HMR1826 and MTH115 in selectively killing tumor cells.

### Examples

### Example 1:

### Generation of a recombinant NDV with transgenes that lead to the expression of an antibody

The oncolytic strain MTH68 of NDV was used to obtain viral RNA. Using RT-PCR several fragments of cDNA were obtained and in a multi-step cloning procedure they were assembled into a full-genome cDNA that was cloned into the vector pX8δT (Schnell et al., 1994) yielding the plasmid pfIMTH68. This vector can be used for transfection in order to rescue recombinant virus from a T7-polymerase expressing cell line.

Two additional transcriptional cassettes were cloned into the full-length genomic plasmid of NDV MTH68 (pfIMTH68) between the genes encoding the F-protein and the HN-protein into the unique Sfil restriction site. The two DNA-oligonucleotides
Sfi fw (5'-aggccttaattaaccgacaacttaagaaaaaatacgggtagaacggcctgag-3', SEQ. ID. NO: 1) and
Sfi back (5'-aggccgttctacccgtattttttcttaagttgtcggttaattaaggcctctc-3', SEQ. ID. NO: 2)
were annealed and subsequently ligated into the Sfil-site of pfIMTH68.

The resulting plasmid pfIMTH68Pac was cut with Pacl and another dsDNA-oligonucleotide consisting of
Asc fw (5'-cgggcgcgccccgacaacttaagaaaaaatacgggtagaacagcagtcttcagtcttaat-3', SEQ.
ID. NO: 3) and Asc back (5'-taagactgaagactgctgttctacccgtattttttcttaagttgtcggggcgcgcccgat-3', SEQ. ID. NO: 4)
was inserted into the Pacl-restriction site thereby destroying one of the flanking Pacl-recognition sites and inserting a unique AscI-site. The resulting plasmid was designated pfIMTH68 Asc Pac. This plasmid has two additional transcriptional cassettes inserted between the genes for the viral F and HN-genes. Both transcriptional cassettes were flanked by identical viral transcriptional start and stop sequences (Fig. 1). Cassette 1 contains a unique PacI restriction site, cassette 2 contains a unique AscI restriction site for the insertion of a transgene cDNA.

The heavy and light chains of a recombinant function blocking therapeutic antibody against the extra-domain B (ED-B) of fibronectin (MOR03257) were cloned into the two additional unique restriction sites AscI and Pacl respectively. The murine Iglambda light chain for the anti-ED-B antibody was inserted into the AscI site. The murine IgG heavy chain for the anti-ED-B antibody was inserted into the Pacl site. The length of the inserts together with the adaptor sequences was adjusted to a base number which was a multiple of six in order to follow the rule of six for the length of the complete genome of the recombinant virus. The resulting plasmid was designated pfIMTH68 murine IgG ED-B (Fig. 2).

Using standard techniques for the rescue of recombinant NDV (transfection of T7-expressing BHK cells), a virus was produced that contained two additional genes coding for the two chains of the anti-ED-B IgG antibody. That virus was called MTH146.

### Example 2:

### Expression of an antibody by recombinant Newcastle Disease Virus. Cells infected with MTH146 produce an antibody that recognizes its antigen ED-B fibronectin (ELISA).

### Materials and Methods:

HT29 (human colon carcinoma) and CHO (chinese hamster ovary) cells were seeded in 6well plates at 4x10⁵ cells/well. The day after, cells were infected in triplicate with a MOI of 0,01 either with virus MTH146 (containing the genes for an anti-ED-B antibody) or with virus MTH115 (containing the gene for an irrelevant secreted transgene [beta-glucuronidase]).

At the time points 0h, 20h, 30h, 2d, 3d, 6d *post infectionem* an aliquot of the cell supernatant was taken and subjected to an antibody titer determination.

The antibody titer was determined by ELISA. Plastic wells were coated with the recombinant antigen ED-B. The tissue culture supernatant containing the antibody or as a standard known amounts of recombinant antibody was added to the wells. After washing, bound antibody was detected with a HRP-coupled secondary antibody raised against murine IgG. Using purified recombinant antibody (expressed by plasmid-transfected cells) as a standard the concentration of the virally produced antibody could be determined.

### Results:

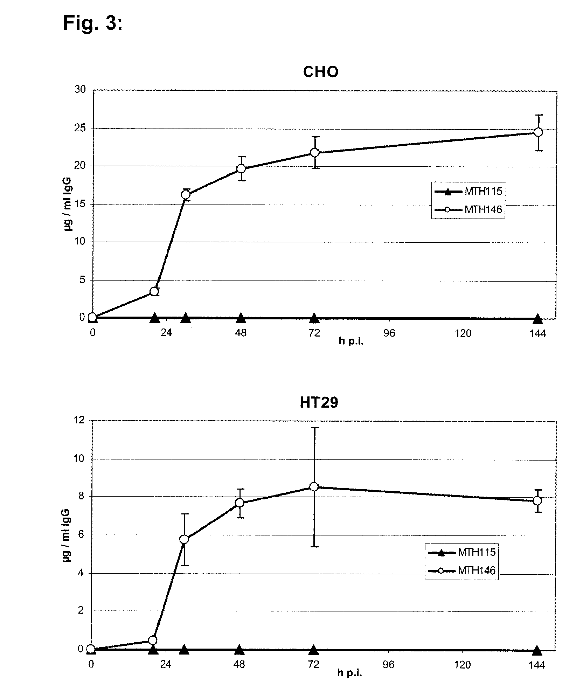

Infection with MTH146 leads to the expression of an antibody into the cell supernatant that specifically binds to its target ED-B (Fig. 3). Infection of the same cells with a virus expressing an irrelevant secreted transgene (MTH115) does not lead to a signal in the ED-B ELISA with the cell supernatant. In HT29 tumor cells the concentration of the antibody in the supernatant reaches titers of ca. 8 µg / ml (binding IgG), which is a sufficient concentration for biological activity. In CHO cells the antibody titer reaches even > 20 µg / ml in the supernatant.

### Example 3:

### Virally expressed antibody binds to its target: Immunofluorescence

### Materials and Methods:

**Murine F9 teratocarcinoma cells were seeded in 6well plates. The cells were** infected either with MTH146 (anti-ED-B antibody) or MTH115 (irrelevant transgene β-glucuronidase) or mock infected. Two days after the infection the cells were fixed with 4% formaldehyde. The cells were washed and bound antibody was visualized by staining with a Cy3-coupled secondary antibody directed against mouse IgG. As a positive control mock-infected cells were incubated prior to fixation for 1 hour with 5 µg/ml recombinant α-ED-B IgG.

### Results:

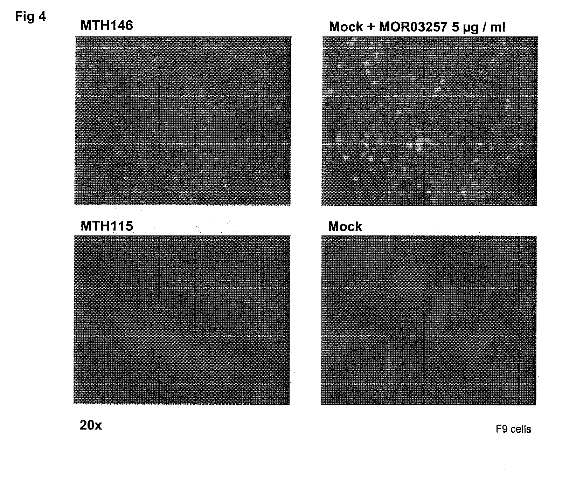

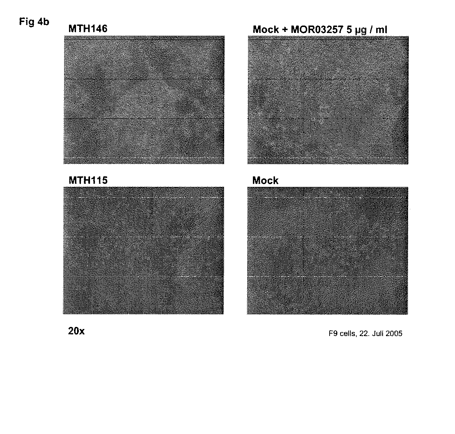

The negative controls do not show a significant staining of the F9 cells. Cells infected with MTH146 show a staining pattern that is indistinguishable from the staining with the recombinant antibody (Fig. 4). Therefore it can be concluded that the viral infection leads to the production of the correct antibody that binds to its antigen like the recombinant antibody. The virally produced antibody concentration is also sufficient to give a staining similar to an antibody concentration of 5 µg/ml. That is consistent with the estimated concentration of the virally expressed antibody, which is in the range of 5 µg/ml.

### Example 4:

### Virally expressed antibody binds to its target: Immunohistochemistry

### Materials and Methods:

Cryopreserved tissue sections (10 µm) of the SK-MEL melanoma grown as xenograft subcutaneously in mice were fixed in cold acetone for 10 min, washed in PBS and stained with antibody. As control served a purified α-ED-B IgG antibody MOR03257 (conc. 5 µg/ml for staining) that was expressed from stable transfected 293 cells. The negative control was incubated with buffer only and no primary antibody. The supernatants of 293 cells infected with recombinant viruses were used for staining as undiluted supernatants. Prior to staining, virus was inactivated by treatment with UV-light. MTH146 infected cells produce the α-ED-B IgG antibody, control infected cells were infected with a recombinant virus that did not produce an antibody as a transgene. The bound antibody was visualized by detection with protein-A-peroxidase and staining with diaminobenzidine as a substrate. The sections were counterstained with hematoxilin QS. Photodocumentation was performed with a Zeiss Axiophot imaging system.

### Results:

No unspecific staining of the cryosections can be observed when incubated without antibody or with a tissue culture supernatant from control-virus-infected 293 cells.

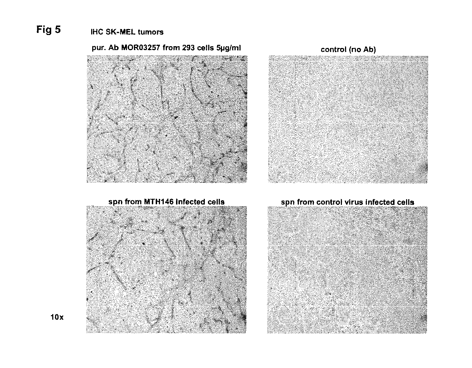

Staining with the purified α-ED-B antibody expressed from stable transfected 293 cells shows a characteristic signal for tumor vasculature where ED-B is present. The incubation with the supernatant from MTH 1 46-infected cells yields an undistinguishable staining pattern (Fig. 5). This demonstrates that the virally expressed antibody is able to specifically bind its target ED-B also in tumor tissue sections and that the concentration in the supernatant of infected cells is sufficient to give a signal comparable to 5 µg/ml purified antibody.

### Example 5:

### Tumor-selective replication of recombinant NDV in vivo

This example demonstrates that intravenous injection of the GFP expressing oncolytic virus MTH87 leads to the expression of GFP in the tumor in a mouse xenograft model.

MiaPaCa pancreas carcinoma xenografts were grown subcutaneously in nude mice. The mice were treated repeatedly (6x) with 1x10⁹ pfu of MTH87 (NDV with GFP as transgene) every other day.

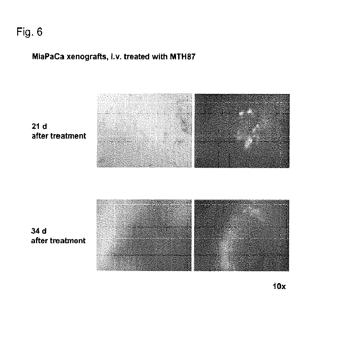

At day 21 and day 34 after the last treatment individual animals were sacrificed and sectioned. Sections of the organs (ca. 2 mm) were directly analysed under a fluorescence microscope to detect GFP-expression (Fig. 6).

Photographs of tumor sections show GFP-expression. No such GFP-expression could be found in any of the following organs: spleen, liver, lung, heart, kidney, intestine, adrenal.

### Reisolation of NDV (MTH87) from organs:

In order to prove that replicating virus was present in the tumor but not in the other organs, slices of the organs (ca. 2 mm thickness) were incubated on top of a monolayer of virus-sensitive HT29 cells in tissue-culture medium. The day after, the HT29 cells were scored for the expression of GFP which indicated infection by MTH87 that originated from the organ/tumor.

Reisolation of MTH87 was only possible from tumor pieces but not from any of the organs tested. Out of 6 tumor pieces from each tumor at least 4 were positive for the virus-reisolation. Negative organs were: spleen, liver, lung, heart, kidney, intestine, adrenal (no other organs were included in the test).

These results document that the recombinant virus MTH87 selectively replicates in tumor tissue and not in other organs. The virus replication is detectable many days after the last intravenous administration of the virus.

### Example 6:

### Generation of an oncolytic NDV that expresses an antibody-effector fusion protein L19-IL2:

The fusion protein L19-IL2 is a antibody targeted cytokine. The antibody fragment L19 is directed against the extradomain B of fibronectin (ED-B) which is expressed mainly surrounding tumor blood vessels but also in the tumor stroma.

The cytokine Interleukin-2 induces proliferation of T-cells and activates NK-cells. Free, non-targeted interleukin-2 (Proleukin) is currently used in low dose application for treatment of Renal cell carcinoma (RCC). The clinical use of Proleukin is limited due to its high systemic toxicity. It has been shown that the targeted delivery of IL-2 by an antibody such as L19 can be used to deliver therapeutic efficacious doses to tumors while maintaining non-toxic systemic levels of IL-2. The tumor specific delivery and expression of L19-IL2 combines the tumorselectivity of the oncolytic virus with the additional tumorselectivity of the L19 antibody fragment and the strong effector activity of IL-2.

### Material and Methods:

### Generation of recombinant NDV expressing the fusion protein L19-IL2

The plasmid pfIMTH68 Pac contains the full genomic sequence of NDV MTH68 plus one additional transcriptional cassette with a unique PacI restriction site. A DNA-transgene coding for the fusion protein L19-IL2 is cloned into the Pacl site. This transgene is composed of a Kozak sequence CCACC, a signal peptide for the extracellular secretion and the cDNA for the fusion protein L19-IL2 (Carnemolla et al., 2002). The total length of the genome is adjusted to be a multiple of 6 to follow the "rule of six" for the length of the viral genome. Recombinant virus is rescued from T7-expressing cells transfected with the full-length viral genomic plasmid containing the gene for L19-IL2 by a standard virus rescue technique. The resulting virus is designated MTH201. The virus is cultivated either in tissue culture or in the allantoic fluid of chicken eggs to produce high titres.

### Murine F9-teratocarcinoma Xenograft experiment

Therapeutic studies are performed in the syngeneic F9 murine teratocarcinoma model. The murine F9-teratocarcinoma model is a rapid-growing syngenic tumor characterized by a high ED-B - fibronectin expression mainly in surrounding tumor blood vessels but also in the tumor stroma.

2 x10⁶ F9 tumor cells in 50% matrigel are implanted s.c. into nude mice strain 129 (clone SvHsd). When tumors reach a size of approximately 20 mm² mice are infected with recombinant NDV MTH201 (encoding the L19-IL2 fusion protein) or control virus MTH87 (encoding GFP as a transgene) at 1x10⁹ pfu at days 1, 3, 5 and 7 intravenously. L19-IL2 (CHO-derived, dimer) and IL2 (PROLEUKINE) are administered as a daily i.v. bolus injection in sterile saline solution over a period of 5 days. All concentrations that are used in animal studies are in the range of 1.8 - 2.16 Mio. IU/kg/day.

After 12 days the mice are sacrificed and the tumor weight is assessed by measurement of the tumor area (product of the longest diameter and its perpendicular) or tumor volume using a caliper.

### Result:

Dimeric L19-IL2 shows a 54.7% tumor weight reduction even at the lowest dose of 1430 IU/g/day corresponding to a low dose regimen used in humans whereas 3- and 6-fold higher doses only slightly improve the therapeutic efficacy of targeted IL2. PROLEUKINE reduces the tumor weight by 50.5% at its highest dose. No therapeutic efficacy is observed at 1430- and 4290 IU/g/day. Best results are obtained in the group of mice treated with NDV MTH201. Tumor weight reduction is above 55%. This can be explained by a combined action of the virus-expressed L19-IL2 and the oncolytic effect of the NDV. Another advantage is that the achieved results are obtained with less administrations of the NDV MTH201 compared to fusion protein alone.

### Example 7:

### Generation of an oncolytic NDV that expresses an ankyrin repeat protein that binds VEGF and is biologically active.

It has been demonstrated that VEGF and its receptor are essential for the growth of colorectal cancers and the formation and growth of colon cancer metastases in the liver in experimental models (Warren et al., 1995) and this principle has now been clinically validated by the systemic use of the VEGF-neutralizing human antibody avastin in patients with metastatic colorectal cancer (Salgaller, 2003).

VEGF (or VPF) is a homodimeric glycoprotein consisting of four isoforms (containing either 121, 165, 189, 206 amino acid residues in the mature monomer) which are generated by alternative splicing of mRNA derived from a single gene. While all forms of VEGF possess a signal sequence only the smaller two species are secreted. In contrast the larger forms are associated with heparin-bound-proteoglycans in the extracellular matrix. VEGF is mitogenic for a variety of large and small vessel endothelial cells, induces the production of tissue factor, collagenase, plasminogen activators, and their inhibitors and stimulates hexose transport in these cells as well. Most important receptors for VEGF are fms-like tyrosine kinase flt1 and KDR (Waltenberger et al., 1994). The Expression of VEGF has been demonstrated in several human cancer lines in vitro and in surgically resected tumors of the human gastrointestinal tract, ovary, brain, kidney, lung, and others. In many experimental tumor models the neutralization of VEGF leads to an efficient and significant growth inhibition (Gerber and Ferrara, 2005).

Here we demonstrate that the tumor targeted delivery of the anti-VEGF ankyrin repeat protein by the recombinant NDV is advantageous for the treatment of this disease. This is shown in an experimental athymic mouse model of colorectal cancer. Typically the liver is the first and most frequent site of metastasis in colorectal cancer. Therefore the potency of the new reagent is also demonstrated in an orthotopic experimental model of liver metastasis.

### Material and Methods:

### Generation of recombinant NDV expressing an ankyrin repeat protein that neutralizes VEGF

An anti-VEGF ankyrin repeat molecule of the type N₃C is selected with standard procedures (Binz et al., 2004). The sequence of the selected ankyrin repeat protein is known. An expression cassette coding for a designed ankyrin repeat protein (dARPIN) with inhibitory activity against VEGF is cloned into the full-length genomic NDV-MTH68 plasmid.

The plasmid pfIMTH68 Pac contains the full genomic sequence of NDV MTH68 plus one additional transcriptional cassette with a unique PacI restriction site. A DNA-transgene coding for the dARPIN agains VEGF is cloned into the Pacl site. This transgene is composed of a Kozak sequence CCACC, a signal peptide for the extracellular secretion and the cDNA for the ankyrin repeat molecule. The total length of the genome is adjusted to be a multiple of 6 to follow the "rule of six" for the length of the viral genome. Recombinant virus is rescued from T7-expressing cells transfected with the full-length viral genomic plasmid containing the gene for the dARPIN agains VEGF by a standard virus rescue technique. The resulting virus is designated MTH268. The virus is cultivated either in tissue culture or in the allantoic fluid of chicken eggs to produce high titres.

### Subcutaneous tumors:

Confluent cultures of LS LiM6 cells are grown in 10 cm² Petri dishes and are harvested by brief trypsinization, (0.05% trypsin/0.02% EDTA in Ca2+/Mg2+ free HBSS) washed several times in Ca2+/Mg2* -free PBS and are resuspended at a final concentration of 5x10⁷ cells in serum free DMEM. The presence of single cells is confirmed by phase contrast microscopy, and cell viability is determined by trypan blue exclusion. Pathogen-free Balb/c NCR-NU athymic mice (3-4 week-old females obtained from Simonson laboratories, Gilroy, Ca) are housed in sterilized cages and injected subcutaneously with 5 x 10⁶ viable tumor cells. Animals are observed daily for tumor growth, and subcutaneous tumors are measured using a caliper every 3 d. On day 1, 3, 5 and 7 after the tumor inoculation groups of five animals are injected intraperitoneally with varying amounts of either anti VEGF mAB 4.6.1 (0 - 200µg per mouse), a control mAb of the same isotype (200 µg/ mouse), with anti-VEGF ankyrin repeat protein (0 - 200 µg/ per mouse), 1x10⁹ pfu NDV MTH268 or 1x10⁹ control NDV MTH87. Prior to high-dose virus treatment the animals are desensitized with the increasing virus doses 1x10⁶ pfu, 1x10⁷ pfu, 1x10⁸ pfu, 5x10⁸ pfu every other day. Tumor volumes are calculated as previously described (Kuan et al., 1987).

### Liver metastases:

H7 cells are grown to confluence and are harvested as described above for subcutaneous injection and resuspended in serum free DMEM at a concentration of 20 x 10⁶ cell/ ml. Athymic mice are anesthetized with methoxyfluorance by inhalation prepared in a sterile fashion, and the spleen is exteriorized through a left flank incision. 2x10⁶ cells in 100 ml are slowly injected into the splenix pulp through a 27-gauge needle over 1 min, followed by splenectomy 1 min later. Experimental animals receive VEGF antibody 4.6.1, control antibody (100 µg/ mouse), anti-VEGF ankyrin repeat protein (100 µg/ ml) by intraperitoneal injection beginning 1 d after splenic-portal injection and every 3 to 4 days thereafter. Alternatively mice are treated intraperitoneally with 1x10⁹ pfu NDV MTH268 or 1x10⁹ control NDV MTH87 at days 1, 3, 5 and 7. All animals are killed when the first mouse appears lethargic and an enlarged liver is palpated (day 28). The livers are excised and weighed, and the metastases are enumerated using a dissecting microscope.

To estimate tumor volume, the diameter of each liver metastases is measured to the nearest millimeter, and the volume of each tumor is calculated by assuming it to be a sphere. The sum of the volumes of all tumors in each liver is determined. The livers of two control animals is nearly replaced by tumor and individual nodules can not be distinguished. Tumor volume is estimated in these two livers as follows: the total liver volume mass is measured by displacement of water in a 20-ml graduated cylinder and the tumor volume is estimated to represent 85% of the liver.

### Result:

**Subcutaneous tumors:**The tumor size of treated animals is monitored and dose-dependent tumor growth inhibition is found for all tested reagents. The tumor size in the animals which received the control mAb is approximately 100 mm³ on day 8, 400 mm³ on day 16 and 900 mm³ on day 22. For the mAb 4.6.1 the measured tumor size at day 22 is 500 mm³ when 10 µg of mAb are injected each time of administration, and 200 mm³ (100 µg), 150 mm³ (50 µg), 100 mm³ (200 µg) respectively. Similar dose dependency is found for the anti-VEGF ankyrin repeat protein when up to 200 µg are injected, with the best result of a tumor size of 100 - 500 mm³ when 200 µg of the reagent are applied. By treatment of athymic mice with the control NDV MTH87 the tumor size at day 22 is above 200 mm³. This antitumor effect is due to the antiproliferative effect caused oncolytic NDV. Best effects are found when athymic mice are infected with NDV MTH268 (expressing the anti-VEGF Ankyrin repeat protein). Measured tumor size is below 100 mm³. This effect is due to the advantageous combination of tumorselective expression of the VEGF-neutralizing ankyrin repeat binding protein and its antiangiogenic effect and the independent antiproliferative effect of the tumorselective NDV on the tumor cells. The tumorselective delivery and expression of the anti-VEGF ankyrin binding protein ensures a steady and high level site-specific expression of the neutralizing protein which overcomes pharmacological limitations of the systemic administered ankyrin repeat protein e.g. such as rapid clearance and non-specific tissue distribution and ensures efficient VEGF neutralisation. This effect is obtained at a reduced number of administrations and therefore a higher convenience for the patient population is expected.

### Liver metastases:

All animals show evidence of hepatic tumors but differ in number, size and weight of liver metastases. A dramatic reduction in comparison to the control mAb treated mice is seen after anti-VEGF treatment. The average number of tumors per liver and the mean estimated tumor volume per liver is 10- and 18-fold lower in the anti-VEGF mAb 4.6.1-treated animals compared to the control antibody treated animals. Similar results are found for the anti-VEGF ankyrin repeat protein treated-mice.The most dramatic reduction is observed in the animals treated with NDV MTH268. Though administered less times the numbers of tumors in the liver is reduced and the tumor volume is smaller when compared to A4.6.1 treated mice.

As for the primary tumor the beneficial effect of NDV MTH268 on the inhibition of formation and growth of liver metastases is due the advantageous combination of tumorselective expression of the VEGF-neutralizing ankyrin repeat binding protein and its antiangiogenic effect and the independent antiproliferative effect of the tumorselective NDV on the tumor cells. The NDV selectively proliferates in these remaining tumor buds and exerts its antiproliferative effect on these tumor cells further decreasing the number of surviving tumor cells and number of detectable metastases.

An even increased therapeutic efficacy on the growth of the primary tumor and the number, size and weight of colorectal liver metastases is seen when a multi-specific ankyrin repeat binding protein is used which contains specificities for VEGF-A, VEGF-C and PDGF.

### Example 8:

### Generation of recombinant NDV expressing an intracellular ankyrin repeat protein that inhibits polo-like kinase activity and inhibits tumor growth

Plk-1 has been shown to be a target for cancer therapy. Expression of Plk-1 is elevated in neoplastic tissues and has a prognostic potential in a broad range of human tumors (see eg. WO2005042505 and references therein).

An anti-human Plk-1 ankyrin repeat molecule of the type N3C is selected with standard procedures (Binz et al., 2004, Amstutz et al., 2005). Binding to Plk-1 is measured in an ELISA with recombinant GST-PIk-1 on glutathion-plates as a substrate. Positive binders are selected and the corresponding genes are cloned into the eukaryotic CMV expression plasmid pcDNA3.1. The plasmids are transfected into Hela and MaTu cells and the cells are subjected to a proliferation assay as described e.g. in WO2005042505. This method is preferred to an in vitro kinase assay in order to be able to also identify binding dARPINS that block function without directly blocking the kinase activity. The dARPIN with the best inhibitory activity of cell proliferation is selected for insertion into the genome of the oncolytic recombinant NDV. The sequence of the selected ankyrin repeat protein comprises 161 amino acids corresponding to 483 bp. An expression cassette coding for the designed ankyrin repeat protein (dARPIN) with inhibitory activity against human Plk-1 is cloned into the full-length genomic NDV-MTH68 plasmid.

The plasmid pfIMTH68 Pac contains the full genomic sequence of NDV MTH68 plus one additional transcriptional cassette with a unique PacI restriction site. A DNA-transgene coding for the dARPIN against PIk-1 is cloned into the Pacl site. This transgene is composed of a Kozak sequence CCACC and the cDNA for the ankyrin repeat molecule. The total length of the genome is adjusted to be a multiple of 6 to follow the "rule of six" for the length of the viral genome. Recombinant virus is rescued from T7-expressing cells transfected with the full-length viral genomic plasmid containing the gene for the dARPIN against PIk-1 by a standard virus rescue technique. The resulting virus is designated MTH261. The virus is cultivated either in tissue culture or in the allantoic fluid of chicken eggs to produce high titres. The virus is purified and concentrated using tangential flow filtration and eluted in buffer with 5% mannitol/1% L-lysine.

### In vivo tumor growth inhibition.

Female NMRI nude mice are injected subcutaneously with 1,5x10⁶ MaTu cells diluted 1:1 (medium : matrigel). When tumors have reached a size of 20-25 mm² (appr. 3 days later) the animals are treated with the recombinant virus. The animals are injected with 200 µl virus suspension (in 5% mannitol/1 % L-lysine buffer) intravenously every other day with the following consecutive doses: 1x10⁶ pfu, 1x10⁷ pfu, 1x10⁸ pfu, 5x10⁸ pfu, 1x10⁹ pfu, 1x10⁹ pfu, 1x10⁹ pfu, 1x10⁹ pfu, 1x10⁹ pfu. The increasing doses at the beginning allow for a desensitisation of the mice against the virus. One group receives only buffer (5% mannitol/1% L-lysine), one group receives recombinant virus MTH87 that expresses a non-therapeutic transgene (GFP) and one group the recombinant oncolytic virus MTH261 expressing the dARPIN that has inhibitory activity against PIk-1. Tumor growth is monitored for three weeks and the tumors are weighed at the end (day 21). Control tumors are in the range of 1,0 g. Tumors treated with MTH87 show a significant weight reduction. Tumors treated with MTH261 are even smaller and show a significant weight reduction in comparison with MTH87. This demonstrates a benefit of the dARPIN expression by the virus compared to the virus treatment alone. The result also proves that an intracellularly active transgene can improve the oncolytic properties of a recombinant oncolytic virus.

### Example 9:

### Generation of an oncolytic NDV that expresses an ankyrin repeat protein that binds HIF1α and is biologically active

Hypoxia in tumors is developed if the tumor cells grow faster than the endothelial cells that form the blood vessel system, leading to a deprivation of oxygen and nutrients in the tumor.

The establishment of hypoxic areas in solid tumors promotes the progression of the tumor development. Oxygen deficiency in tumor cells leads to genetic instability, followed by a selection process, ending up in tumor cells with reduced apoptotic potential and increased resistance against conventional therapies, like chemotherapy and radiation. As a consequence hypoxia in tumors contributes to a more malignant phenotype.

The hypoxia-inducible transcription factor HIF-1α is mainly responsible for the activation of most genes in tumor cells under hypoxic conditions. HIF-1α induced gene products are regulating processes like angiogenesis, glucose metabolism, cell growth and oxygen transport. Examples of upregulated genes by HIF-1α are glucose transporter 1 and 3, adenylate-kinase 3, lactate dehydrogenase, VEGF, FIt-1 and others.

The heterodimeric transcription factor HIF-1α is composed of a HIF-1α and a HIF-1β subunit. The HIF-1α-subunit and the HIF-1β-subunits are constitutively expressed. But HIF-1α is in contrast to HIF-1β immediately degraded under normoxic conditions. In a normal oxygen milieu the HIF-1β subunit is recognised and marked for proteasomal degradation by the Von-Hippel-Lindau protein (pVHL), which is part of an E3 ubiquitin ligase complex. Under hypoxic condition the HIF-1α-subunit is no more recognised by the pVHL and the protein is immediately transported into the nucleus. In the nucleus a dimerisation with the H IF-1β-subunit takes place and DNA binding to a HRE (hypoxia reponse element) leads to specific gene induction.

In the described experiment it is demonstrated that intracellular targeting of the HIF-1α-transcription factor by an anti HIF-1α ankyrin repeat protein expressed by a recombinant NDV in a solid tumor model is superior over NDV treatment alone and application of recombinant purified anti HIF-1α ankyrin repeat protein alone. In vivo efficacy is proven in a prostate cancer xenograft model in nude mice.

### Material and Methods

### Generation of recombinant NDV expressing an ankyrin repeat protein that binds HIF-1 α

An anti-HIF-1α ankyrin repeat molecule of the type N3C is selected with standard procedures (Binz et al., 2004). The sequence of the selected ankyrin repeat protein is known. An expression cassette coding for a designed ankyrin repeat protein (dARPIN) with inhibitory activity against HIF-1α is cloned into the full-length genomic NDV-MTH68 plasmid.

The plasmid pfIMTH68 Pac contains the full genomic sequence of NDV MTH68 plus one additional transcriptional cassette with a unique PacI restriction site. A DNA-transgene coding for the dARPIN against HIF1α is cloned into the Pacl site. This transgene is composed of a Kozak sequence CCACC and the cDNA for the ankyrin repeat molecule. The total length of the genome is adjusted to be a multiple of 6 to follow the "rule of six" for the length of the viral genome. Recombinant virus is rescued from T7-expressing cells transfected with the full-length viral genomic plasmid containing the gene for the dARPIN agains HIF-1α by a standard virus rescue technique. The resulting virus is designated MTH288. The virus is cultivated either in tissue culture or in the allantoic fluid of chicken eggs to produce high titers.

### PC-3 xenograft model:

PC-3 xenografts are passaged in vivo in athymic female or male nude mice. Xenografts are established by subcutaneous (sc) injection of 5 x 10⁵ PC-3 cells per animal. After the third passage tumors are cut into 2 mm³ pieces. Subcutaneous inocculation of nonnecrotic tumor tissues in nude mice is performed using sterile stainless steel needles. Mice are randomly allocated to various treatment groups, when the tumor volume reaches an average size of 150-200 mm³.

On day 0 after the group allocation the mice are treated every other day with increasing doses of MTH288 (encoding anti HIF-1α dARPIN) or MTH87 (control virus encoding GFP as a transgene). Prior to high dose virus treatment, virus doses of 10⁶ PFU, 10⁷ PFU, 10⁸ PFU and 5x10⁸ PFU are given intravenously for desensitization against NDV. After desensitization the highest dose of 10⁹ PFU is applied three times. The control group is treated intravenously with purified anti HIF-1α ankyrin repeat protein (200 µg/per mouse). Application of the anti HIF-1α ankyrin repeat protein starts at the time point of the first virus injection and is also repeated every other day as long as the virus treatment is done. A third control group is treated with PBS alone. On day 20 after the first treatment animals are observed for tumor growth and subcutaneous tumors are measured using a caliper.

### Results:

On day 20 after first treatment the tumors of PBS injected animals show in average a volume of 1500 mm³. The second control group treated only with anti HIF-1α ankyrin repeat protein displays a slight tumor growth reduction. Animals receiving the NDV MTH87 show a strong tumor reduction on day. The best tumor growth inhibition on day 20 is seen in mice injected with the NDV MTH288 expressing the anti HIF-1α ankyrin repeat protein. The inhibition of tumor growth after treatment with MTH288 is significantly better than after the other treatments.

This result demonstrates that intracellular expression of an ankyrin repeat protein against HIF-1α by NDV is advantageous over NDV treatment alone or over application of recombinant purified anti HIF-1α ankyrin repeat protein.

It is expected that the viral expression of a secreted anti HIF-1α ankyrin repeat protein fused to a cell penetrating peptide (e.g. tat peptide, oligoarginine peptides, AntP peptide, VP22 peptide, penetratin, transportan (for review see (*Ford et al., 2001*) enhances the therapeutic effect. This strategy allows the targeting of tumor cells with the anti HIF-1α ankyrin repeat fusion protein that are themselves not infected with NDV.

A virus that expresses simultaneously transgenes for two separate dARPINS - one intracellular against HIV-1α and one secreted against interleukin-8 - is expected to have an increased anti-tumor activity due to blockade of two compensatory pathways (Mizukami et al., 2005).

### Example 10

### MTH1151 leads to expression of β-glucuronidase in infected cells

### Materials and Methods:

Hela cells were plated in 6well dishes. After reaching confluency cells were infected with very low MOI = 0.00001 of MTH87 and MTH115. At the indicated time points aliquots of the cell supernatant were taken and frozen at -20°C. 72 hours after the infection the activity of β-glucuronidase was measured simultaneously in all supernatants in a MUG-assay.

### MUG-assay for the quantification of β-glucuronidase activity

MU (4-methylumbelliferon) and MUG (4-methylumbelliferyl-β-D-glucuronide) are from Sigma. MU is used to produce a standard curve. Virus containing samples are UV-radiated for 30 min to inactivate virus prior to the MUG-assay. Samples are incubated in a 25 mM sodium-acetate buffer pH 5.6 with 10 µM MUG for 60 min at 37°C. The reaction is stopped by addition of 200 mM glycin buffer pH 10.4. Fluorescence is measured with excitation at 320 nm and emission at 460 nm in a fluorescence photometer. From the fluorescence emission the specific activity in nmol per ml per hour is calculated.

### Results:

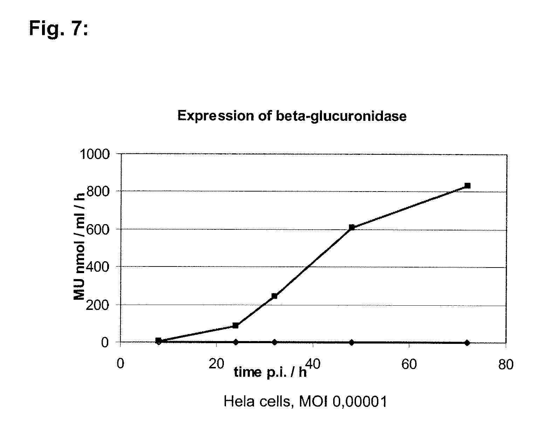

The infection with MTH115 leads to a time-dependent increase in the expression of β-glucuronidase, which is a result of the viral replication (Figure 7). Although MTH87 eventually kills the cells and thus liberates endogenous β-glucuronidase from the cells the resulting activity in the supernatant is negligable. MTH115 infection clearly achieves a massive production of the transgene. The CPE by MTH115 is comparable to that of MTH87 and MTH68 (wt), so the expression of β-glucuronidase by the virus does neither attenuate replication of the virus nor decrease the cytopathogenicity of the virus.

### Example 11

### Quantification of transgene expression by MTH115 in various cells

### Materials and Methods:

Cells were seeded in 96well plates. The number of cells was chosen to reach confluency one day after plating:
- Hela: 15 000 Cells / well
- HT29: 30 000 Cells / well
- Fibroblasts: 10 000 Cells / well
- HDMVEC: 20 000 Cells / well

One day after seeding cells were infected with the virus MTH115 at an MOI of 0.01. In detail cells were washed with PBS, inoculated with 100 µl virus-suspension at 37°C, virus was aspirated and 200 µl warm medium was added to the cells.

At the indicated time points the supernatants were taken off the cells and subject to a beta-glucuronidase MUG-assay. For each time point triplicate wells were analysed.

### Results:

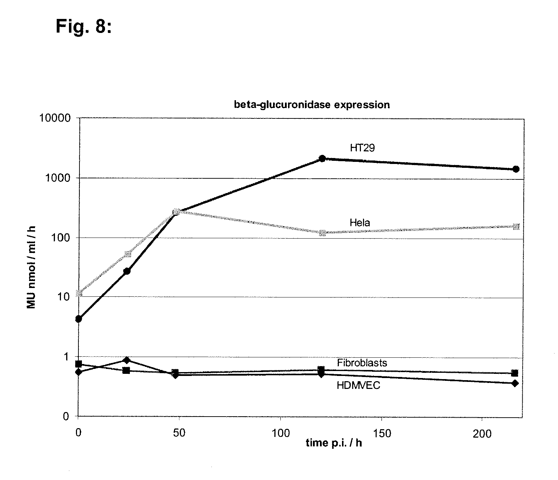

The results are summarized in Figure 8. In the normal cells HDMVEC and fibroblasts no transgene expression can be detected. Also no cytopathic effect was observed (not shown). In the tumor cells that are susceptible for the virus Hela and HT29 there is a massive production of the transgene beta-glucuronidase whose activity can be measured in the supernatant. Hela cells which are killed by the virus within 48 hours produce relatively lower levels of the transgene. HT29 cells that die only after 5 days at the virus doses used, produce even higher activity of beta-glucuronidase, about 10fold more than Hela cells and more than 1000fold over background.

### Example 12

### NDV-beta-glucuronidase and HMR1826 synergistically kill cells that are resistant to either treatment alone

### Materials and Methods:

Cells were plated on day 1 in 24well dishes. On day 2 the cells were infected with NDV-beta-glucuronidase (MTH115) at an MOI of 0,001. On day 3 the cells were treated either with doxorubicin at 1 µM or the Doxorubicin-glucuronide HMR1826 at 1 µM. On day 8 the cells were washed, fixed and stained with Giemsa and photographs of the wells were taken.

### Results:

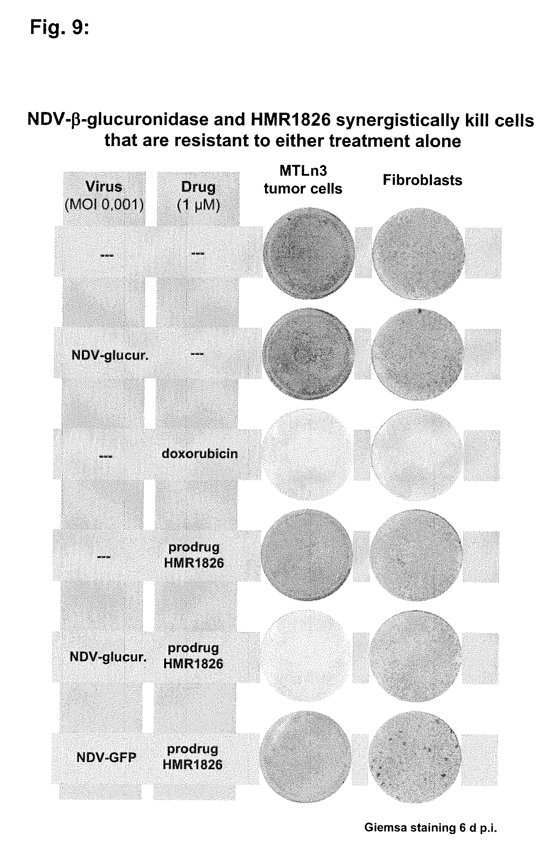

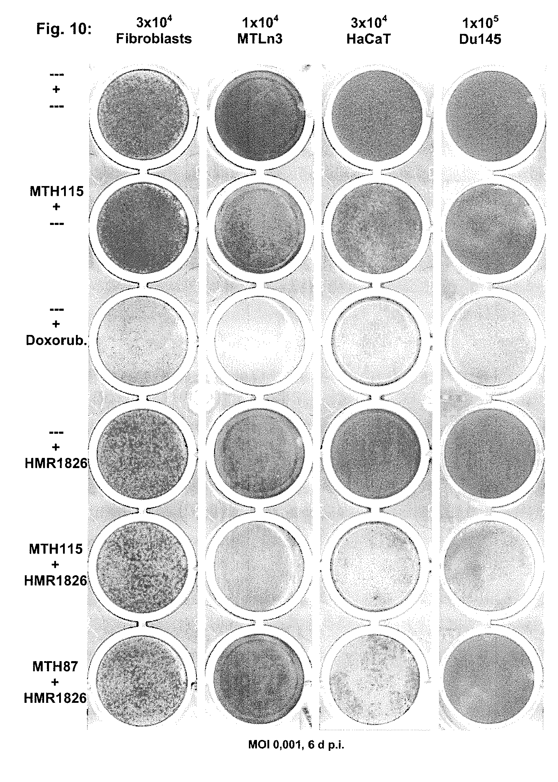

The results are summarized in Figure 9 and Figure 10. Doxorubicin alone kills the cells at the given concentration. The prodrug at the same concentration is not toxic to the cells. The virus alone at the given MOI is also not toxic because tumor cells were selected that have a degree of resistance to the virus treatment. The combination of both glucuronidase-expressing virus and the prodrug is able to kill cells that are not killed by either treatment alone. A control virus (that expresses GFP instead of beta-glucuronidase) does not show any synergy with the prodrug treatment.

### References

Adams, G.P. and Weiner, L.M. (2005) Monoclonal antibody therapy of cancer. Nat Biotechnol, 23, 1147-1157
Alexander, D.J. (1988) Newcastle disease. Kluwer Academic Publishers, Norwell, Massachusetts
Allen, T.M. (2002) Ligand-targeted therapeutics in anticancer therapy. Nat Rev Cancer, 2, 750-763
Amstutz, P., Binz, H.K., Parizek, P., Stumpp, M.T., Kohl, A., Grutter, M.G., Forrer, P. and Pluckthun, A. (2005) Intracellular kinase inhibitors selected from combinatorial libraries of designed ankyrin repeat proteins. J Biol Chem, 280, 24715-24722
Antoniw, P., Springer, C.J., Bagshawe, K.D., Searle, F., Melton, R.G., Rogers, G.T., Burke, P.J. and Sherwood, R.F. (1990) Disposition of the prodrug 4-(bis (2-chloroethyl) amino) benzoyl-L-glutamic acid and its active parent drug in mice. Br J Cancer, 62, 909-914
Bagshawe, K.D., Springer, C.J., Searle, F., Antoniw, P., Sharma, S.K., Melton, R.G. and Sherwood, R.F. (1988) A cytotoxic agent can be generated selectively at cancer sites. Br J Cancer, 58, 700-703
Batra, J.K., Fitzgerald, D.J., Chaudhary, V.K. and Pastan, I. (1991) Single-chain immunotoxins directed at the human transferrin receptor containing Pseudomonas exotoxin A or diphtheria toxin: anti-TFR(Fv)-PE40 and DT388-anti-TFR(Fv). Mol Cell
Biol, 11, 2200-2205
Batra, J.K., Kasprzyk, P.G., Bird, R.E., Pastan, I. and King, C.R. (1992) Recombinant anti-erbB2 immunotoxins containing Pseudomonas exotoxin. Proc Natl Acad Sci U S A, 89, 5867-5871
Bell, J.C., Garson, K.A., Lichty, B.D. and Stojdl, D.F. (2002) Oncolytic viruses: programmable tumor hunters. Curr Gene Ther, 2, 243-254
Binz, H.K., Amstutz, P., Kohl, A., Stumpp, M.T., Briand, C., Forrer, P., Grutter, M.G. and Pluckthun, A. (2004) High-affinity binders selected from designed ankyrin repeat protein libraries. Nat Biotechnol, 22, 575-582
Bosslet, K., Czech, J., Lorenz, P., Sedlacek, H.H., Schuermann, M. and Seemann, G. (1992) Molecular and functional characterisation of a fusion protein suited for tumor specific prodrug activation. BrJ J Cancer, 65, 234-238
Carnemolla, B., Borsi, L., Balza, E., Castellani, P., Meazza, R., Berndt, A., Ferrini, S., Kosmehl, H., Neri, D. and Zardi, L. (2002) Enhancement of the antitumor properties of interleukin-2 by its targeted delivery to the tumor blood vessel extracellular matrix. Blood, 99, 1659-1665
Chaudhary, V.K., Gallo, M.G., FitzGerald, D.J. and Pastan, I. (1990) A recombinant single-chain immunotoxin composed of anti-Tac variable regions and a truncated diphtheria toxin. Proc Natl Acad Sci U S A, 87, 9491-9494
Chaudhary, V.K., Queen, C., Junghans, R.P., Waldmann, T.A., FitzGerald, D.J. and Pastan, I. (1989) A recombinant immunotoxin consisting of two antibody variable domains fused to Pseudomonas exotoxin. Nature, 339, 394-397
Chiocca, E.A. (2002) Oncolytic viruses. Nat Rev Cancer, 2, 938-950
Csatary, L.K., Gosztonyi, G., Szeberenyi, J., Fabian, Z., Liszka, V., Bodey, B. and Csatary, C.M. (2004) MTH-68/H oncolytic viral treatment in human high-grade gliomas. J Neurooncol, 67, 83-93
De Lorenzo, C., Arciello, A., Cozzolino, R., Palmer, D.B., Laccetti, P., Piccoli, R. and D'Alessio, G. (2004) A fully human antitumor immunoRNase selective for ErbB-2-positive carcinomas. Cancer Res, 64, 4870-4874
Engel-Herbert, I., Werner, O., Teifke, J.P., Mebatsion, T., Mettenleiter, T.C. and Romer-Oberdorfer, A. (2003) Characterization of a recombinant Newcastle disease virus expressing the green fluorescent protein. J Virol Methods, 108, 19-28
Ferrara, N., Hillan, K.J., Gerber, H.P. and Novotny, W. (2004) Discovery and development of bevacizumab, an anti-VEGF antibody for treating cancer. Nat Rev Drug Discov, 3, 391-400
Ford, K.G., Souberbielle, B.E., Darling, D. and Farzaneh, F. (2001) Protein transduction: an alternative to genetic intervention? Gene Ther, 8, 1-4
Georgakis, G.V., Li, Y., Humphreys, R., Andreeff, M., O'Brien, S., Younes, M., Carbone, A., Albert, V. and Younes, A. (2005) Activity of selective fully human agonistic antibodies to the TRAIL death receptors TRAIL-R1 and TRAIL-R2 in primary and cultured lymphoma cells: induction of apoptosis and enhancement of doxorubicin-and bortezomib-induced cell death. Br J Haematol, 130, 501-510
Gerber, H.P. and Ferrara, N. (2005) Pharmacology and pharmacodynamics of bevacizumab as monotherapy or in combination with cytotoxic therapy in preclinical studies. Cancer Res, 65, 671-680
Hanahan, D. and Weinberg, R.A. (2000) The hallmarks of cancer. Cell, 100, 57-70
Herbst, R.S. and Langer, C.J. (2002) Epidermal growth factor receptors as a target for cancer treatment: the emerging role of IMC-C225 in the treatment of lung and head and neck cancers. Semin Oncol, 29, 27-36
Hogbom, M., Eklund, M., Nygren, P.A. and Nordlund, P. (2003) Structural basis for recognition by an in vitro evolved affibody. Proc Natl Acad Sci U S A, 100, 3191-3196
Huang, Z., Elankumaran, S., Yunus, A.S. and Samal, S.K. (2004) A recombinant Newcastle disease virus (NDV) expressing VP2 protein of infectious bursal disease virus (IBDV) protects against NDV and IBDV. J Virol, 78, 10054-10063
Huang, Z., Krishnamurthy, S., Panda, A. and Samal, S.K. (2001) High-level expression of a foreign gene from the most 3'-proximal locus of a recombinant Newcastle disease virus. J Gen Virol, 82, 1729-1736
Kreitman, R.J., Chaudhary, V.K., Waldmann, T.A., Hanchard, B., Cranston, B., FitzGerald, D.J. and Pastan, I. (1993) Cytotoxic activities of recombinant immunotoxins composed of Pseudomonas toxin or diphtheria toxin toward lymphocytes from patients with adult T-cell leukemia. Leukemia, 7, 553-562
Kreitman, R.J. and Pastan, I. (1995) Importance of the glutamate residue of KDEL in increasing the cytotoxicity of Pseudomonas exotoxin derivatives and for increased binding to the KDEL receptor. Biochem J, 307 (Pt 1), 29-37
Krishnamurthy, S., Huang, Z. and Samal, S.K. (2000) Recovery of a virulent strain of newcastle disease virus from cloned cDNA: expression of a foreign gene results in growth retardation and attenuation. Virology, 278, 168-182
Kuan, S.F., Byrd, J.C., Basbaum, C.B. and Kim, Y.S. (1987) Characterization of quantitative mucin variants from a human colon cancer cell line. Cancer Res, 47, 5715-5724
Ladner, R.C. and Ley, A.C. (2001) Novel frameworks as a source of high-affinity ligands. Curr Opin Biotechnol, 12, 406-410
Lamb, R.A., Kolakofsky, D. (2001) Paramyxoviridae: The Viruses And Their Replication. In Fields, B.N. (ed.), Virology. Lippincott Williams & Wilkins, pp. 1305-1485
Lorence, R.M., Pecora, A.L., Major, P.P., Hotte, S.J., Laurie, S.A., Roberts, M.S., Groene, W.S. and Bamat, M.K. (2003) Overview of phase I studies of intravenous administration of PV701, an oncolytic virus. Curr Opin Mol Ther, 5, 618-624
Mizukami, Y., Jo, W.S., Duerr, E.M., Gala, M., Li, J., Zhang, X., Zimmer, M.A., lliopoulos, O., Zukerberg, L.R., Kohgo, Y., Lynch, M.P., Rueda, B.R. and Chung, D.C. (2005) Induction of interleukin-8 preserves the angiogenic response in HIF-1alpha-deficient colon cancer cells. Nat Med, 11, 992-997
Nakaya, T., Cros, J., Park, M.S., Nakaya, Y., Zheng, H., Sagrera, A., Villar, E., Garcia-Sastre, A. and Palese, P. (2001) Recombinant Newcastle disease virus as a vaccine vector. J Virol, 75, 11868-11873
Noonberg, S.B. and Benz, C.C. (2000) Tyrosine kinase inhibitors targeted to the epidermal growth factor receptor subfamily: role as anticancer agents. Drugs, 59, 753-767
Nord, K., Gunneriusson, E., Uhl inverted question marken, M. and Nygren, P.A. (2000) Ligands selected from combinatorial libraries of protein A for use in affinity capture of apolipoprotein A-1 M and taq DNA polymerase. J Biotechnol, 80, 45-54
Roffler, S.R., Wang, S.M., Chern, J.W., Yeh, M.Y. and Tung, E. (1991) Anti-neoplastic glucuronide prodrug treatment of human tumor cells targeted with a monoclonal antibody-enzyme conjugate. Biochem Pharmacol, 42, 2062-2065
Romer-Oberdorfer, A., Mundt, E., Mebatsion, T., Buchholz, U.J. and Mettenleiter, T.C. (1999) Generation of recombinant lentogenic Newcastle disease virus from cDNA. J Gen Virol, 80 (Pt 11), 2987-2995
Rowlinson-Busza, G., Bamias, A., Krausz, T. and Epenetos, A.A. (1992) Antibody guided enzyme nitrile therapy (Agent): in vitro cytotoxicity and in vivo tumor localisation. In Epenetos, a. (ed.), Monoclonal Antibodies 2. Applications in Clinical Oncology. Chapman and Hall, London, pp. 111-118
Russell, S.J. (2002) RNA viruses as virotherapy agents. Cancer Gene Ther, 9, 961-966
Salgaller, M.L. (2003) Technology evaluation: bevacizumab, Genentech/Roche. Curr Opin Mol Ther, 5, 657-667
Schnell, M.J., Mebatsion, T. and Conzelmann, K.K. (1994) Infectious rabies viruses from cloned cDNA. Embo J, 13, 4195-4203
Sinkovics, J.G. and Horvath, J.C. (2000) Newcastle disease virus (NDV): brief history of its oncolytic strains. J Clin Virol, 16, 1-15
Skerra, A. (2000) Lipocalins as a scaffold. Biochim Biophys Acta, 1482, 337-350
Stojdl, D.F., Lichty, B.D., tenOever, B.R., Paterson, J.M., Power, A.T., Knowles, S., Marius, R., Reynard, J., Poliquin, L., Atkins, H., Brown, E.G., Durbin, R.K., Durbin, J.E., Hiscott, J. and Bell, J.C. (2003) VSV strains with defects in their ability to shutdown innate immunity are potent systemic anti-cancer agents. Cancer Cell, 4, 263-275
Vogelstein, B. and Kinzler, K.W. (2004) Cancer genes and the pathways they control. Nat Med, 10, 789-799
Waltenberger, J., Claesson-Welsh, L., Siegbahn, A., Shibuya, M. and Heldin, C.H. (1994) Different signal transduction properties of KDR and FIt1, two receptors for vascular endothelial growth factor. J Biol Chem, 269, 26988-26995
Wang, S.M., Chern, J.W., Yeh, M.Y., Ng, J.C., Tung, E. and Roffler, S.R. (1992) Specific activation of glucuronide prodrugs by antibody-targeted enzyme conjugates for cancer therapy. Cancer Res, 52, 4484-4491
Warren, R.S., Yuan, H., Matli, M.R., Gillett, N.A. and Ferrara, N. (1995) Regulation by vascular endothelial growth factor of human colon cancer tumorigenesis in a mouse model of experimental liver metastasis. J Clin Invest, 95, 1789-1797
Zewe, M., Rybak, S.M., Dubel, S., Coy, J.F., Welschof, M., Newton, D.L. and Little, M. (1997) Cloning and cytotoxicity of a human pancreatic RNase immunofusion. Immunotechnology, 3, 127-136
Zhao, H. and Peeters, B.P. (2003) Recombinant Newcastle disease virus as a viral vector: effect of genomic location of foreign gene on gene expression and virus replication. J Gen Virol, 84, 781-788

## Claims

1. The recombinant oncolytic Newcastle disease virus (NDV) strain MTH-68 comprising a nucleic acid with a transgene, wherein the nucleic acid of this transgene codes for the enzyme beta-glucuronidase, beta-galactosidase, beta-glucosidase, carboxypeptidase, beta-lactamase or the immune-stimulatory protein with cytokine activity IL-2, IL-12, TNF-alpha, IFN-beta or GM-CSF, or the ankyrin repeat protein with inhibitory activity against HIF1α, or ankyrin repeat protein with inhibitory activity against soluble VEGF-R, or the ankyrin repeat molecule of N3C with inhititory activity against mitotic kinases Plk-1 that has a therapeutic activity when expressend by the virus-infected tumour cell.

2. A nucleocapsid of the NDV of claim 1.

3. A genome of the NDV of claims 1 and 2.

4. A DNA molecule encoding the genome and/or antigenome of the NDV of claims 1 and 2.

5. The DNA molecule of claim 4 operatively linked to a transcriptional control sequence.

6. A cell comprising the NDV of claims 1 and 2, a virus genome of claim 3 or a DNA molecule of claim 4.

7. A pharmaceutical composition comprising the NDV of claims 1 and 2, a virus genome of claim 3 or a DNA molecule of claim 4 or 5 as an active ingredient optionally together with pharmaceutically acceptable carriers, diluents and/or adjuvants.

8. The pharmaceutical composition of claim 7 for the prevention and/or treatment of cancer.

9. The use of a composition of claim 7 or 8, for the production of a medicament in a pharmaceutically effective amount for the prevention and/or treatment of cancer.


<110>  Bayer Schering Pharma Aktiengesellschaft

<120>  Recombinant Newcastle Disease Virus

<130>  53277A

<150>  EP04090432.8
<151>  2004-11-11

<160>  5

<170>  PatentIn version 3.3

<210>  1
<211>  52
<212>  DNA
<213>  Artificial

<220>
<223>  Sfi fw, DNA-Oligonucleotide

<400>  1
aggccttaat taaccgacaa cttaagaaaa aatacgggta gaacggcctg ag          52


<210>  2
<211>  52
<212>  DNA
<213>  Artificial

<220>
<223>  Sfi back, DNA-Oligonucleotide

<400>  2
aggccgttct acccgtattt tttcttaagt tgtcggttaa ttaaggcctc tc          52


<210>  3
<211>  60
<212>  DNA
<213>  Artificial

<220>
<223>  Asc fw, DNA-Oligonucleotide

<400>  3
cgggcgcgcc ccgacaactt aagaaaaaat acgggtagaa cagcagtctt cagtcttaat    60


<210>  4
<211>  60
<212>  DNA
<213>  Artificial

<220>
<223>  Asc back, DNA-Oligonucleotide

<400>  4
taagactgaa gactgctgtt ctacccgtat tttttcttaa gttgtcgggg cgcgcccgat    60


<210>  5

<211>  318

<212>  DNA

<213>  Artificial

<220>
<223>  Intergenic region between F and HN of plasmid pflMTH68_Asc_Pac,
       DNA-Oligonucleotide

<400>  5
atgcagatga gaggcagagg tatccccaat agcaatctgt gtgtcaattc tggcagcctg      60

ttaatcagaa gaattaagaa aaaactaccg gatgtaggtg aacaaaaggg aatatacggg     120

tagaacggcc tgagaggcct taatcgggcg cgccccgaca acttaagaaa aaatacgggt     180

agaacagcag tcttcagtct taattaaccg acaacttaag aaaaaatacg ggtagaacgg     240

cctgagaggc cacccctcaa tcgggagcca ggccccacta cgtccgctct accgcaacac     300

caacagcagt cttcagtc                                                   318